# EUROPEAN PATENT SPECIFICATION

(11) **EP 2 411 372 B1**
(45) Date of publication and mention of the grant of the patent: **23.07.2014**
(21) Application number: 10711968.7
(22) Date of filing: 24.03.2010
(51) Int. Cl.: C07D 239/54, C07D 311/08

(54) **PROCESS FOR PREPARING AN ANTIVIRAL COMPOUND**
VERFAHREN ZUR HERSTELLUNG EINER ANTIVIRALEN VERBINDUNG
PROCÉDÉ DE PRÉPARATION D'UN COMPOSÉ ANTIVIRAL

(30) Priority: 24.03.2009 US 162705 P; 23.03.2010 US 316713 P
(43) Date of publication of application: 01.02.2012
(73) Proprietor: AbbVie Bahamas Ltd., New Providence, Nassau (BS)
(72) Inventor: NAPIER, James, J., Antioch IL 60002 (US); CALIFANO, Jean-Christophe, Whitefish Bay WI 53217 (US); BECKER, Calvin, L., Kenosha, Wisconsin 53142 (US); YU, Su, Lake Bluff, Illinois 60044 (US)
(74) Representative: Kingsbury, Oliver William
(86) International application number: PCT/US2010/028433
(87) International publication number: WO 2010/111348

(56) References cited:
- WO-A1-2007/138242
- WO-A1-2009/039127
- WO-A1-2009/039135
- MICHELI F ET AL: "Phenylethynyl-pyrrolo[1,2-a]pyrazine: A new potent and selective tool in the mGluR5 antagonists arena" BIOORGANIC & MEDICINAL CHEMISTRY LETTERS, PERGAMON, ELSEVIER SCIENCE, GB LNKD- DOI:10.1016/J.BMCL.2008.02.024, vol. 18, no. 6, 15 March 2008 (2008-03-15) , pages 1804-1809, XP025694971 ISSN: 0960-894X [retrieved on 2008-02-14]

## Description

### CROSS-REFERENCE TO RELATED PATENT APPLICATIONS

This patent application claims priority to U.S. Provisional Patent Application No. 61/162,705 (filed March 24, 2009) and U.S. Provisional Patent Application No. 61/316,713 (filed March 23, 2010). The entire text of those applications is incorporated by reference into this patent application.

### FIELD OF THE INVENTION

This invention is directed to: (a) processes for preparing a compound and salts thereof that, *inter alia,* are useful for inhibiting hepatitis C virus (HCV); (b) intermediates useful for the preparation of the compound and salts; (c) pharmaceutical compositions comprising the compound or salts; and (d) methods of use of such compositions.

### BACKGROUND OF THE INVENTION

Hepatitis C is a blood-borne, infectious, viral disease that is caused by a hepatotropic virus called HCV. At least six different HCV genotypes (with several subtypes within each genotype) are known to date. In North America, HCV genotype 1a predominates, followed by HCV genotypes 1b, 2a, 2b, and 3a. In the United States, HCV genotypes 1, 2, and 3 are the most common, with about 80% of the hepatitis C patients having HCV genotype 1. In Europe, HCV genotype 1b is predominant, followed by HCV genotypes 2a, 2b, 2c, and 3a. HCV genotypes 4 and 5 are found almost exclusively in Africa. As discussed below, the patient's HCV genotype is clinically important in determining the patient's potential response to therapy and the required duration of such therapy.

An HCV infection can cause liver inflammation (hepatitis) that is often asymptomatic, but ensuing chronic hepatitis can result in cirrhosis of the liver (fibrotic scarring of the liver), liver cancer, and/or liver failure. The World Health Organization estimates that about 170 million persons worldwide are chronically infected with HCV, and from about three to about four million persons are newly infected globally each year. According to the Centers for Disease Control and Prevention, about four million people in the United States are infected with HCV. Co-infection with the human immunodeficiency virus (HIV) is common, and rates of HCV infection among HIV positive populations are higher.

There is a small chance of clearing the virus spontaneously, but the majority of patients with chronic hepatitis C will not clear it without treatment. Indications for treatment typically include proven HCV infection and persistent abnormal liver function tests. There are two treatment regimens that are primarily used to treat hepatitis C: monotherapy (using an interferon agent - either a "conventional" or longer-acting pegylated interferon) and combination therapy (using an interferon agent and ribavirin). Interferon, which is injected into the bloodstream, works by bolstering the immune response to HCV; and ribavirin, which is taken orally, is believed to work by preventing HCV replication. Taken alone, ribavirin does not effectively suppress HCV levels, but an interferon/ribavirin combination is more effective than interferon alone. Typically, hepatitis C is treated with a combination of pegylated interferon alpha and ribavirin for a period of 24 or 48 weeks, depending on the HCV genotype.

The goal of treatment is sustained viral response -- meaning that HCV is not measurable in the blood after therapy is completed. Following treatment with a combination of pegylated interferon alpha and ribavirin, sustained cure rates (sustained viral response) of about 75% or better occur in people with HCV genotypes 2 and 3 in 24 weeks of treatment, about 50% in those with HCV genotype 1 with 48 weeks of treatment, and about 65% in those with HCV genotype 4 in 48 weeks of treatment.

Treatment may be physically demanding, particularly for those with prior history of drug or alcohol abuse, because both interferon and ribavirin have numerous side effects. Common interferon-associated side effects include flu-like symptoms, extreme fatigue, nausea, loss of appetite, thyroid problems, high blood sugar, hair loss, and skin reactions at the injection site. Possible serious interferon-associated side effects include psychoses *(e.g.,* suicidal behavior), heart problems *(e.g.,* heart attack, low blood pressure), other internal organ damage, blood problems (*e.g*., blood counts falling dangerously low), and new or worsening autoimmune disease (*e.g*., rheumatoid arthritis). Ribavirin-associated side effects include anemia, fatigue, irritability, skin rash, nasal stuffiness, sinusitis, and cough. Ribavirin can also cause birth defects, so pregnancy in female patients and female partners of male patients must be avoided during treatment and for six months afterward.

Some patients do not complete treatment because of the serious side effects discussed above; other patients (non-responders) continue to have measurable HCV levels despite treatment; and yet other patients (relapsers) "clear" the virus during therapy, but the virus returns sometime after completion of the treatment regimen. Thus, there continues to be a need for alternative compositions and methods of treatment (used either in combination with or in lieu of an interferon agent and/or ribavirin) to alleviate the symptoms of hepatitis C, thereby providing partial or complete relief. This invention provides processes for preparing one such compound -- (E)-N-(4-(3-tert-butyl-5-(2,4-dioxo-3,4-dihydro- pyrimidin-1(2H)-yl)-2-methoxystyryl)phenyl)methanesulfonamide) (compound **I**) -- and salts thereof.

### SUMMARY OF THE INVENTION

This invention is directed to a process for preparing (E)-N-(4-(3-tert-butyl-5-(2,4-dioxo-3,4-dihydropyrimidin-1(2H)-yl)-2-methoxystyryl)phenyl)methanesulfonamide (compound **I**) or a salt thereof, wherein the process comprises reducing N-(4-((3-tert-butyl-5-(2,4-dioxo-3,4-dihydro pyrimidin-1(2H)-yl)-2-methoxy- phenyl)ethynyl)phenyl)methanesulfonamide (compound **6**).

This invention also is directed to compound **I** and salt thereof prepared by the above process.

This invention also is directed to a process for preparing compound **6.**

This invention also is directed to compound **6.**

This invention also is directed to various intermediates useful for preparing compound **6** as well as to processes for preparing those intermediates.

This invention also is directed to compositions (including pharmaceutical compositions) that comprise compound **I** or salt thereof that are prepared by the above processes. Optionally, the compositions can comprise one or more additional therapeutic agents.

This invention also is directed to methods of use of the above compositions to, for example, inhibit replication of a ribonucleic acid (RNA) virus (including HCV) or treat a disease treatable by inhibiting HCV RNA polymerase (including hepatitis C).

Further benefits of Applicants' invention will be apparent to one skilled in the art from reading this patent application.

### DETAILED DESCRIPTION OF THE INVENTION

This detailed description is intended only to acquaint others skilled in the art with Applicants' invention, its principles, and its practical application so that others skilled in the art may adapt and apply the invention in its numerous forms, as they may be best suited to the requirements of a particular use. This description and its specific examples are intended for purposes of illustration only. This invention, therefore, is not limited to the embodiments described in this patent application, and may be variously modified.

### A. Processor Preparing (E)-N-(4-(3-tert-butyl-5-(2,4-dioxo-3,4-dihydropyrimidin-1 (2H)-yl)-2-methoxystyryl)phenyl)methanesulfonamide (compound I).

As discussed above, this invention is directed, in part, to a process for preparing (E)-N-(4-(3-tert-butyl-5-(2,4-dioxo-3,4-dihydropyrimidin-1(2H)-yl)-2-methoxystyryl)phenyl)methanesulfonamide (compound **I**) or a salt thereof. The process comprises reducing N-(4-((3-tert-butyl-5-(2,4-dioxo-3,4-dihydropyrimidin-1(2H)-yl)-2-methoxyphenyl) ethynyl)phenyl)methanesulfonamide (compound **6**):

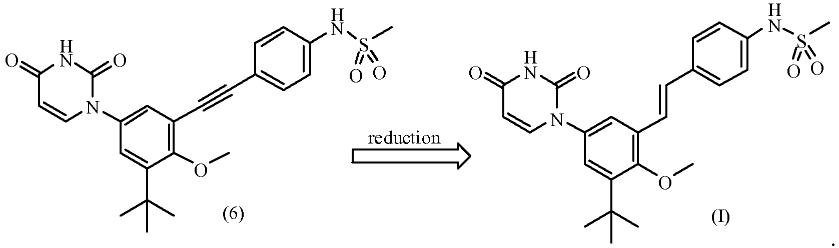

The preparation of starting compound **6** is discussed below.

Compound **6** is reduced using a reducing agent.

In some embodiments, the reducing agent is hydrogen source.

In some embodiments, the reducing agent is silane. Suitable silanes include, for example, triethylsilane, phenylsilane, diphenylsilane, tripropylsilane, triphenylsilane, tribenzylsilane, 1,1,1,3,5,5,5-heptamethyltrisiloxane, tributylsilane, di-tert-butylsilane, diethyloxysilane, dimethoxysilane, phenyldiethoxyethylsilane, dimethylsilane, halosilane, and tris(trimethylsilyl)silane. In some embodiments, the silane is triethylsilane. In other embodiments, the silane is phenylsilane. In yet other such embodiments, the silane is diphenylsilane. In yet other embodiments, the silane is tripropylsilane. In yet other embodiments, the silane is triphenylsilane. In yet other embodiments, the silane is tribenzylsilane. In yet other embodiments, the silane is 1,1,1,3,5,5,5-heptamethyltrisiloxane. In further embodiments, the silane is tributylsilane. In yet further embodiments, the silane is di-tert-butylsilane. In yet further embodiments, the silane is diethyloxysilane. In yet further embodiments, the silane is dimethoxysilane. In yet other embodiments, the silane is phenyldiethoxyethylsilane. In yet further embodiments, the silane is dimethylsilane. In yet further embodiments, the silane is halosilane. In yet further embodiments, the silane is tris(trimethylsilyl)silane.

In some embodiments, the reducing agent is disilane. Suitable disilanes include, for example, hexamethyldisilane, hexaphenyldisilane, 1,2-diphenyltetramethyldisilane, 1,2-dimethyl-1,1,2,2-tetra-phenyldisilane, 1,1,2,2-tetramethyldisilane, 1,2-diethoxy-1,1,2,2-tetramethyldisilane, 1,2-dimethoxy-1,1,2,2-tetramethyldisilane, and hexamethoxydisilane. In some embodiments, the disilane is hexamethyldisilane. In other embodiments, the disilane is hexaphenyldisilane. In yet other embodiments, the disilane is 1,2-diphenyltetramethyldisilane. In yet other embodiments, the disilane is 1,2-dimethyl-1,1,2,2,-tetraphenyldisilane. In further embodiments, the disilane is 1,1,2,2-tetramethyldisilane. In yet further embodiments, the disilane is 1,2-diethoxy-1,1,2,2-tetramethyldisilane. In yet further embodiments, the disilane is 1,2-dimethoxy-1,1,2,2-tetramethyldisilane. In yet further embodiments, the disilane is hexamethoxydisilane.

Compound **6** typically is reduced in the presence of catalyst. In some embodiments, the catalyst is transition metal catalyst. In some such embodiments, the catalyst is palladium catalyst. Suitable palladium catalysts include, for example, tetrakis(triphenylphosphine)palladium (0), dichlorobis(tri-o-tolylphosphine)palladium (II), palladium (II) acetate, [1,1'-bis(diphenylphosphino) ferrocene]dichloropalladium (II), tris(dibenzylideneacetone) dipalladium (0), dichloro(dibenzylideneacetone)dipalladium (II), dichlorotris(cyclohexylphosphine) palladium (II), dichlorobis(triphenylphosphine) palladium (II), and chloro(η3-allyl)palladium(II) dimer-triphenylphosphine. In some embodiments, the catalyst is tetrakis(triphenylphosphine) palladium (0). In other embodiments, the catalyst is dichlorobis(tri-o-tolylphosphine) palladium (II). In other embodiments, the catalyst is palladium (II) acetate. In other embodiments, the catalyst is [1,1'-bis(diphenylphosphino)ferrocene] dichloro palladium (II). In yet other embodiments, the catalyst is tris(dibenzylideneacetone) dipalladium (0). In yet other embodiments, the catalyst is dichloro(dibenzylideneacetone) dipalladium (II). In further embodiments, the catalyst is dichlorotris(cyclohexylphosphine) palladium (II). In yet further embodiments, the catalyst is dichlorobis(triphenylphosphine) palladium (II). In yet further embodiments, the catalyst is chloro(η3-allyl)palladium(II) dimer-triphenylphosphine.

In some embodiments, compound **6** is reduced in the presence of solvent. Suitable solvents include, for example, terahydrofuran, dimethylformamide, dimethylacetamide, and N-methylpyrrolidone. In some embodiments, the solvent is terahydrofuran. In some such other embodiments, the solvent is dimethylformamide. In yet other such embodiments, the solvent is dimethylacetamide.

In yet other such embodiments, the solvent is N-methylpyrrolidone.

In some embodiments, compound **6** is reduced at a temperature of from about 20 °C to about 130 °C. In some such embodiments, the temperature is from about 65 °C to about 85 °C.

In some embodiments, compound **6** is reduced in inert atmosphere. In some such embodiments, the inert atmosphere is provided by nitrogen. In some other such embodiments, the inert atmosphere is provided by argon.

### B. Processor Preparing N-(4-((3-tert-butyl-5-(2,4-dioxo-3,4-dihydropyrimidin-1(2H)-yl)-2-methoxyphenyl) ethynyl)phenyl)methanesulfonamide (Compound 6).

In some embodiments, compound **6** is prepared by reacting 1-(3-tert-butyl-5-ethynyl-4-methoxyphenyl)pyrimidine-2,4(1H,3H)-dione (compound **4**) with compound **2** selected from the group consisting of N-(4-iodophenyl)methanesulfonamide (compound **2a**), N-(4-bromophenyl) methanesulfonamide (compound **2b**), N-(4-chlorophenyl)methanesulfonamide (compound **2c**), N-(4-[(arylsulfonyl)oxy]phenyl)methanesulfonamide (compound **2d**), and N-(4-[(perfluoroalkylsulfonyl) oxy]phenyl)methanesulfonamide (compound **2e)** as follows:

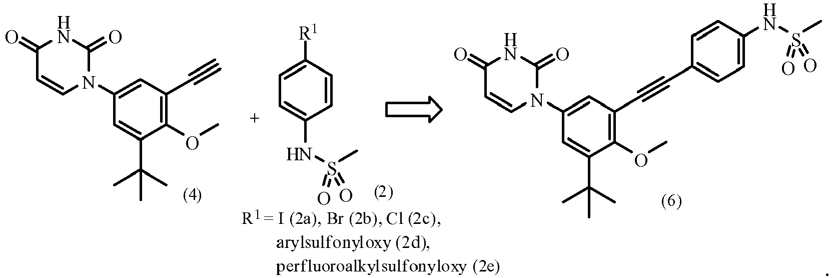

The preparations of starting compound **2** and compound **4** are discussed below.

In some embodiments, compound **4** and compound **2** are reacted in a Sonogashira reaction.

In some embodiments, compound **4** and compound **2** are reacted in the presence of palladium catalyst. Suitable palladium catalyst include, for example, tetrakis(triphenylphosphine)palladium (0), dichlorobis(tri-o-tolylphosphine)palladium (II), palladium (II) acetate, [1,1'-bis(diphenylphosphino) ferrocene]dichloropalladium (II), tris(dibenzylideneacetone) dipalladium (0), dichloro(dibenzylidene acetone)dipalladium (II), and dichlorobis(triphenylphosphine) palladium (II). In some embodiments, the palladium catalyst is tetrakis(triphenylphosphine) palladium (0). In other embodiments, the palladium catalyst is dichlorobis(tri-o-tolylphosphine)palladium (II). In other embodiments, the palladium catalyst is palladium (II) acetate. In yet other embodiments, the palladium catalyst is [1,1'-bis(diphenylphosphino) ferrocene]dichloropalladium (II). In yet other embodiments, the palladium catalyst is tris(dibenzylideneacetone) dipalladium (0). In yet other embodiments, the palladium catalyst is dichloro(dibenzylideneacetone)dipalladium (II). In further embodiments, the palladium catalyst is dichlorobis(triphenylphosphine) palladium (II).

In some embodiments, compound **4** and compound **2** are reacted in the presence of copper catalyst (in addition to palladium catalyst). Suitable copper catalysts include, for example, copper (I) oxide and halide salts of copper (I). Suitable halide salts of copper (I) include, for example, copper (I) iodide, copper (I) bromide, copper (I) iodide dimethyl sulfide, and copper (I) chloride. In some embodiments, the copper catalyst is copper (I) oxide. In other embodiments, the copper catalyst is copper (I) iodide. In other embodiments, the copper catalyst is copper (I) bromide. In yet other embodiments, the copper catalyst is copper (I) iodide dimethyl sulfide. In yet other embodiments, the copper catalyst is copper (I) chloride.

In some embodiments, compound **4** and compound **2** are reacted in the presence of base. Suitable bases include, for example, triethylamine, diisopropylethyl amine, sodium carbonate, cesium carbonate, and potassium carbonate. In some embodiments, the base is triethylamine. In other embodiments, the base is diisopropylethyl amine. In yet other embodiments, the base is sodium carbonate. In yet other embodiments, the base is cesium carbonate. In yet other embodiments, the base is potassium carbonate.

In some embodiments, compound **4** and compound **2** are reacted in the presence of solvent. Suitable solvents include, for example, terahydrofuran, dimethylformamide, dimethoxyethane, dimethylacetamide, N-methylpyrrolidone, and toluene. In some embodiments, the solvent is terahydrofuran. In other embodiments, the solvent is dimethylformamide. In other embodiments, the solvent is dimethoxyethane. In other embodiments, the solvent is dimethylacetamide. In yet other embodiments, the solvent is N-methylpyrrolidone. In yet other embodiments, the solvent is toluene.

In some embodiments, compound **4** and compound **2** are reacted in inert atmosphere. In some such embodiments, the inert atmosphere is provided by nitrogen. In other such embodiments, the inert atmosphere is provided by argon.

In some embodiments, compound **4** and compound **2** are reacted at a temperature of from about 20 °C to about 130 °C. In some such embodiments, the temperature is from about 20 °C to about 30 °C.

In some embodiments, compound **2** is compound **2a.** In other embodiments, compound **2** is compound **2b.** In yet other embodiments, compound **2** is compound **2c.** In yet other embodiments, compound **2** is compound **2d.** In some such embodiments, aryl is phenyl in compound **2d.** In some other such embodiments, aryl is naphthyl in compound **2d.** In yet other embodiments, compound **2** is compound **2e.** In some such embodiments, perfluoroalkyl is perfluoro-C₁-C₆-alkyl in compound **2e.**

In some embodiments, compound **4** and compound **2** are reacted as follows:

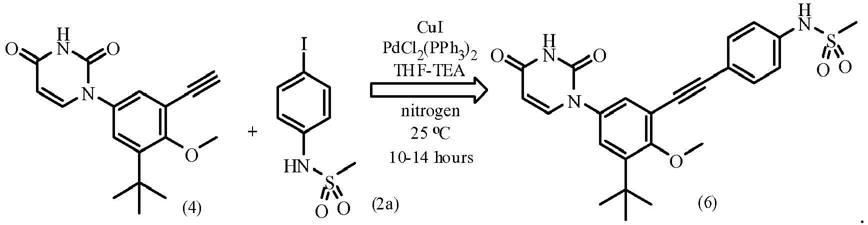

### C. N-(4-((3-tert-butyl-5-(2,4-dioxo-3,4-dihydropyrimidin-1(2H)-yl)-2-methoxyphenyl) ethynyl)phenyl)methanesulfonamide (compound 6).

This invention is directed, in part, to N-(4-((3-tert-butyl-5-(2,4-dioxo-3,4-dihydropyrimidin-1(2H)-yl)-2-methoxyphenyl) ethynyl)phenyl)methanesulfonamide (compound **6**) or a salt thereof:

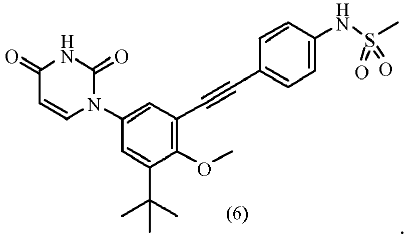

### D. Preparation of compound 2.

As discussed above, compound **2** selected from the group consisting of N-(4-iodophenyl) methanesulfonamide (compound **2a**), N-(4-bromophenyl) methanesulfonamide (compound **2b**), N-(4-chlorophenyl)methanesulfonamide (compound **2c**), N-(4-[(arylsulfonyl)oxy]phenyl)methanesulfonamide (compound **2d**), and N-(4-[(perfluoroalkylsulfonyl) oxy]phenyl)methanesulfonamide (compound **2e**). It is Applicants' understanding that compound **2** is commercially available. It may also be prepared by one skilled in the art without undue experimentation.

In some embodiments, compound **2** is prepared by reacting methanesulfonyl chloride (MsCl) with compound **1** selected from the group consisting of 4-iodoaniline (compound **1a**), 4-bromoaniline (compound **1b**), 4-chloroaniline (compound **1c**), and 4-hydroxyaniline (compound **1d**). In the case of compound **1d,** the subsequent formation of compound **2d** (R¹ = arylsufonyloxy) and compound **2e** (R¹ = perfluoroalkylsulfonyloxy) can be achieved by, for example, sulfonylation of compound **1d** with aryl-SO₂Cl or perfluoroalkyl-SO₂Cl, respectively:

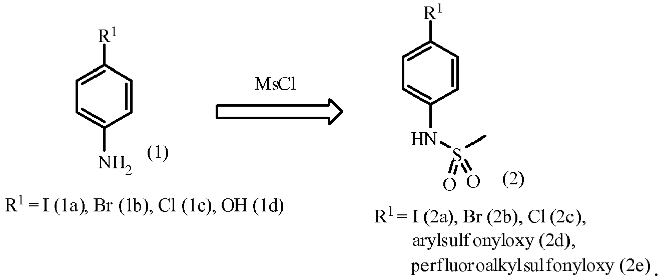

It is Applicants' understanding that compound **1** is commercially available. It may also be prepared by one skilled in the art without undue experimentation.

### E. Preparation of 1-(3-tert-butyl-5-ethynyl-4-methoxyphenyl)pyrimidine-2,4(1H,3H)-dione (compound 4).

In some embodiments, compound **4** is prepared by reacting ethynyltrimethylsilane (≡TMS) (compound **7**) with compound **3** selected from the group consisting of 1-(3-tert-butyl-5-iodo-4-methoxyphenyl) pyrimidine-2,4(1H,3H)-dione (compound **3a**), 1-(3-bromo-5-tert-butyl-4-methoxyphenyl)pyrimidine-2,4(1H,3H)-dione (compound **3b**), and 1-(3-tert-butyl-5-chloro-4-methoxyphenyl)pyrimidine-2,4(1H,3H)-dione (compound **3c**) thus forming 1-(3-tert-butyl-4-methoxy-5-((trimethylsilyl)ethynyl)phenyl)pyrimidine-2,4(1H,3H)-dione (compound **8**): 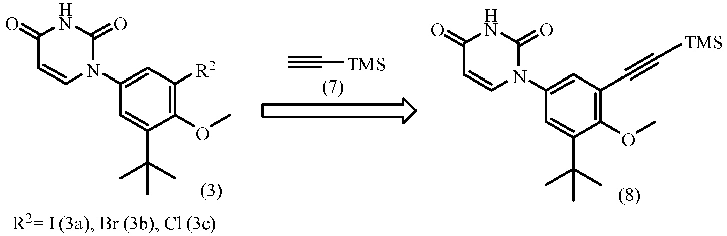 and then removing the trimethylsilyl (TMS) group from the formed 1-(3-tert-butyl-4-methoxy-5-((trimethylsilyl)ethynyl)phenyl)pyrimidine-2,4(1H,3H)-dione (compound 8):

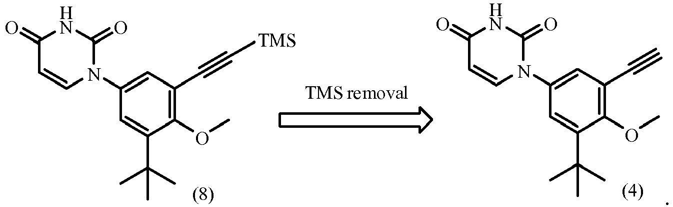

The preparation of compound **3** is discussed below. Compound **7** can be purchased from a commercial source or can be prepared by a person of ordinary skill in the art.

In some embodiments, compound **3** and compound **7** are reacted in the presence of palladium catalyst. Suitable palladium catalyst include, for example, tetrakis(triphenylphosphine)palladium (0), dichlorobis(tri-o-tolylphosphine)palladium (II), palladium (II) acetate, [1,1'-bis(diphenylphosphino) ferrocene]dichloropalladium (II), tris(dibenzylideneacetone) dipalladium (0), dichloro(dibenzylideneacetone)dipalladium (II), and dichlorobis(triphenylphosphine) palladium (II). In some embodiments, the palladium catalyst is tetrakis(triphenylphosphine) palladium (0). In other embodiments, the palladium catalyst is dichlorobis(tri-o-tolylphosphine)palladium (II). In other embodiments, the palladium catalyst is palladium (II) acetate. In yet other embodiments, the palladium catalyst is [1,1'-bis(diphenylphosphino) ferrocene]dichloropalladium (II). In yet other embodiments, the palladium catalyst is tris(dibenzylideneacetone) dipalladium (0). In yet other embodiments, the palladium catalyst is dichloro(dibenzylideneacetone)dipalladium (II). In further embodiments, the palladium catalyst is dichlorobis(triphenylphosphine) palladium (II).

In some embodiments, compound **3** and compound **7** are reacted in the presence of copper catalyst (in addition to the palladium catalyst). Suitable copper catalysts include, for example, copper (I) oxide and halide salts of copper (I). Suitable halide salts of copper (I) include, for example, copper (I) iodide, copper (I) bromide, copper (I) iodide dimethyl sulfide, and copper (I) chloride. In some embodiments, the copper catalyst is copper (I) oxide. In other embodiments, the copper catalyst is copper (I) iodide. In other embodiments, the copper catalyst is copper (I) bromide. In yet other embodiments, the copper catalyst is copper (I) iodide dimethyl sulfide. In yet other embodiments, the copper catalyst is copper (I) chloride.

In some embodiments, compound **3** and compound **7** are reacted in the presence of base. Suitable bases include, for example, triethylamine, diisopropylethyl amine, sodium carbonate, cesium carbonate, and potassium carbonate. In some embodiments, the base is triethylamine. In other such embodiments, the base is diisopropylethyl amine. In other embodiments, the base is sodium carbonate. In yet other embodiments, the base is cesium carbonate. In further embodiments, the base is potassium carbonate.

In some embodiments, compound **3** and compound **7** are reacted in the presence of solvent. Suitable solvents include, for example, terahydrofuran, dimethylformamide, dimethoxyethane, N-methylpyrrolidone, dimethylacetamide, and toluene. In some embodiments, the solvent is terahydrofuran. In other embodiments, the solvent is dimethylformamide. In yet other embodiments, the solvent is dimethoxyethane. In yet other embodiments, the solvent is N-methylpyrrolidone. In yet other embodiments, the solvent is dimethylacetamide. In further embodiments, the solvent is toluene.

In some embodiments, compound **3** and compound **7** are reacted in inert atmosphere. In some such embodiments, the inert atmosphere is provided by nitrogen. In some other such embodiments, the inert atmosphere is provided by argon.

In some embodiments, compound **3** and compound **7** are reacted at a temperature of from about 20 °C to about 130 °C. In some such embodiments, the temperature is from about 20 °C to about 50 °C. In other such embodiments, the temperature is from about 20 °C to about 30 °C. In yet other such embodiments, the temperature is from about 40 °C to about 50 °C.

In some embodiments, compound **3** is compound **3a.** In some other embodiments, compound **3** is compound **3b.** In yet other embodiments, compound **3** is compound **3c.**

In some embodiments, the TMS group is removed by reacting compound **8** with base. Suitable bases, include, for example, tribasic potassium phosphate, potassium carbonate, potassium methoxide, potassium hydroxide, sodium carbonate, and sodium methoxide. In some embodiments, the base is tribasic potassium phosphate. In other embodiments, the base is potassium carbonate. In yet other embodiments, the base is potassium methoxide. In further embodiments, the base is potassium hydroxide. In yet other such embodiments, the base is sodium carbonate. In further embodiments, the base is sodium methoxide.

In some embodiments, the TMS group is removed by reacting compound **8** with fluoride source. Suitable fluoride sources include, for example, potassium fluoride, tetrabutylammonium fluoride, pyridinium fluoride, and triethylammonium fluoride. In some embodiments, the fluoride source is potassium fluoride. In other embodiments, the fluoride source is tetrabutylammonium fluoride. In yet other embodiments, the fluoride source is pyridinium fluoride. In further embodiments, the fluoride source is triethylammonium fluoride.

In some embodiments, TMS group removal is conducted in the presence of solvent. Suitable solvents include, for example, terahydrofuran, dimethylformamide, dimethoxyethane, N-methylpyrrolidone, methanol, ethanol, and isopropanol. In some embodiments, the solvent is terahydrofuran. In other embodiments, the solvent is dimethylformamide. In other embodiments, the solvent is dimethoxyethane. In yet other embodiments, the solvent is N-methylpyrrolidone. In yet other embodiments, the solvent is methanol. In yet other embodiments, the solvent is ethanol. In further embodiments, the solvent is isopropanol.

### F. 1-(3-tert-butyl-4-methoxy-5-((trimethylsilyl)ethynyl)phenyl)pyrimidine-2,4(1H,3H)-dione (compound 8).

This invention is directed, in part, to 1-(3-tert-butyl-4-methoxy-5-((trimethylsilyl)ethynyl) phenyl)pyrimidine- 2,4(1H,3H)-dione (compound **8**) or a salt thereof:

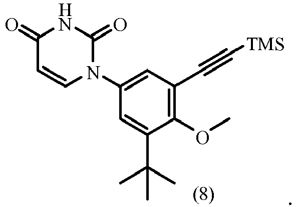

### G. 1-(3-tert-butyl-5-ethynyl-4-methoxyphenyl)pyrimidine-2,4(1H,3H)-dione (compound 4).

This invention is directed, in part, to 1-(3-tert-butyl-5-ethynyl-4-methoxyphenyl)pyrimidine-2,4(1H,3H)-dione (compound **4**) or a salt thereof:

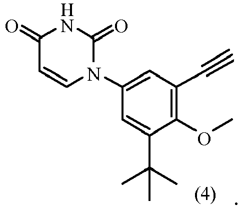

### H. Alternative Processor Preparing N-(4-((3-tert-butyl-5-(2,4-dioxo-3,4-dihydropyrimidin-1(2H)-yl)-2-methoxyphenyl) ethynyl)phenyl)methanesulfonamide (compound 6).

In some embodiments, compound **6** is prepared by reacting N-(4-ethynylphenyl)methane sulfonamide (compound **5**) with compound 3 selected from the group consisting of 1-(3-tert-butyl-5-iodo-4-methoxyphenyl)pyrimidine-2,4(1H,3H)-dione (compound **3a**), 1-(3-bromo-5-tert-butyl-4-methoxyphenyl)pyrimidine-2,4(1H,3H)-dione (compound **3b**), and 1-(3-tert-butyl-5-chloro-4-methoxyphenyl)pyrimidine-2,4(1H,3H)-dione (compound **3c**) thus forming N-(4-((3-tert-butyl-5-(2,4-dioxo-3,4-dihydropyrimidin-1(2H)-yl)-2-methoxyphenyl) ethynyl)phenyl) methanesulfonamide (compound **6**):

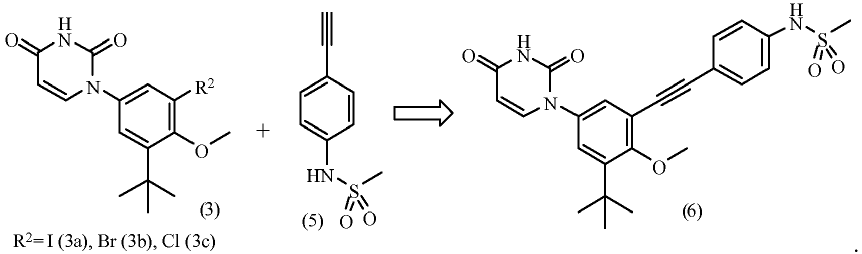

The preparations of compound **3** and compound **5** are discussed below.

In some embodiments, compound **3** and compound **5** are reacted in the presence of palladium catalyst. Suitable palladium catalyst include, for example, tetrakis(triphenylphosphine)palladium (0), dichlorobis(tri-o-tolylphosphine)palladium (II), palladium (II) acetate, [1,1'-bis(diphenylphosphino) ferrocene]dichloropalladium (II), tris(dibenzylideneacetone) dipalladium (0), dichloro(dibenzylideneacetone)dipalladium (II), and dichlorobis(triphenylphosphine) palladium (II). In some embodiments, the palladium catalyst is tetrakis(triphenylphosphine) palladium (0). In other embodiments, the palladium catalyst is dichlorobis(tri-o-tolylphosphine)palladium (II). In other embodiments, the palladium catalyst is palladium (II) acetate. In yet other such embodiments, the palladium catalyst is [1,1'-bis(diphenylphosphino) ferrocene]dichloropalladium (II). In yet other embodiments, the palladium catalyst is tris(dibenzylideneacetone) dipalladium (0). In yet other embodiments, the palladium catalyst is dichloro(dibenzylideneacetone)dipalladium (II). In further embodiments, the palladium catalyst is dichlorobis(triphenylphosphine) palladium (II).

In some embodiments, compound **3** and compound **5** are reacted in the presence of copper catalyst (in addition to palladium catalyst). Suitable copper catalysts include, for example, copper (I) oxide and halide salts of copper (I). Suitable halide salts of copper (I) include, for example, copper (I) iodide, copper (I) bromide, copper (I) iodide dimethyl sulfide, and copper (I) chloride. In some embodiments, the copper catalyst is copper (I) oxide. In other embodiments, the copper catalyst is copper (I) iodide. In other embodiments, the copper catalyst is copper (I) bromide. In yet other embodiments, the copper catalyst is copper (I) iodide dimethyl sulfide. In yet other embodiments, the copper catalyst is copper (I) chloride.

In some embodiments, compound **3** and compound **5** are reacted in the presence of base. Suitable bases include, for example, triethylamine, diisopropylethyl amine, sodium carbonate, cesium carbonate, and potassium carbonate. In some embodiments, the base is triethylamine. In other embodiments, the base is diisopropylethyl amine. In yet other embodiments, the base is sodium carbonate. In yet other embodiments, the base is cesium carbonate. In further embodiments, the base is potassium carbonate.

In some embodiments, compound **3** and compound **5** are reacted in the presence of solvent. Suitable solvents include, for example, terahydrofuran, dimethylformamide, dimethoxyethane, N-methylpyrrolidone, dimethylacetamide, and toluene. In some embodiments, the solvent is terahydrofuran. In other embodiments, the solvent is dimethylformamide. In yet other embodiments, the solvent is dimethoxyethane. In yet other embodiments, the solvent is N-methylpyrrolidone. In yet other embodiments, the solvent is dimethylacetamide. In further embodiments, the solvent is toluene.

In some embodiments, compound **3** and compound **5** are reacted in inert atmosphere. In some such embodiments, the inert atmosphere is provided by nitrogen. In some other such embodiments, the inert atmosphere is provided by argon.

In some embodiments, compound **3** is compound **3a.** In some other embodiments, compound **3** is compound **3b.** In yet other embodiments, compound **3** is compound **3c.**

### I. Preparation of compound 3.

The preparation of compound **3** is described in Example 1 below as well as in International Patent Application No. PCT/US08/76576.

### J. Preparation of N-(4-ethynylphenyl)methanesulfonamide (compound 5).

In some embodiments, N-(4-ethynylphenyl)methanesulfonamide (compound **5**) is prepared by reacting ethynyltrimethylsilane (≡TMS) (compound 7) with compound **2** thus forming N-(4-((trimethylsilyl)ethynyl)phenyl) methanesulfonamide (compound **9**): 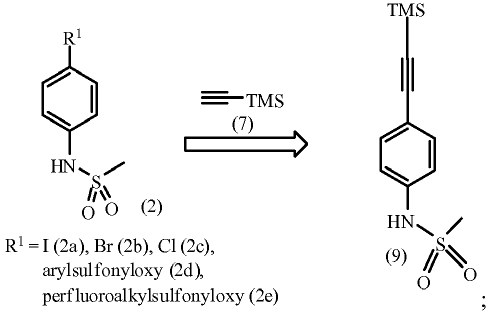 and then removing the trimethylsilyl (TMS) group from the formed N-(4-((trimethylsilyl)ethynyl) phenyl)methanesulfonamide (compound **9**):

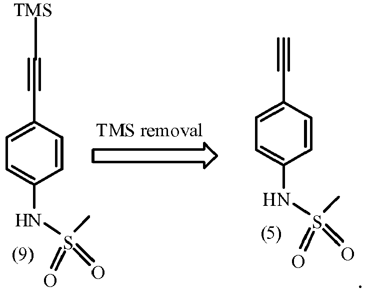

The preparation of compound **2** is discussed above. Compound **7** is commercially available, or, alternatively, can be prepared by a person of ordinary skill in the art.

In some embodiments, compound **2** and compound **7** are reacted in the presence of palladium catalyst. Suitable palladium catalysts include, for example, tetrakis(triphenylphosphine)palladium (0), dichlorobis(tri-o-tolylphosphine)palladium (II), palladium (II) acetate, [1,1'-bis(diphenylphosphino) ferrocene]dichloropalladium (II), tris(dibenzylideneacetone) dipalladium (0), dichloro(dibenzylideneacetone)dipalladium (II), and dichlorobis(triphenylphosphine) palladium (II). In some embodiments, the palladium catalyst is tetrakis(triphenylphosphine) palladium (0). In other embodiments, the palladium catalyst is dichlorobis(tri-o-tolylphosphine)palladium (II). In yet other embodiments, the palladium catalyst is palladium (II) acetate. In yet other embodiments, the palladium catalyst is [1,1'-bis(diphenylphosphino) ferrocene]dichloropalladium (II). In yet other embodiments, the palladium catalyst is tris(dibenzylideneacetone) dipalladium (0). In yet other embodiments, the palladium catalyst is dichloro(dibenzylideneacetone)dipalladium (II). In yet other embodiments, the palladium catalyst is dichlorobis(triphenylphosphine) palladium (II).

In some embodiments, compound **2** and compound **7** are reacted in the presence of copper catalyst (in addition to palladium catalyst). Suitable copper catalysts include, for example, copper (I) oxide and halide salts of copper (I). Suitable halide salts of copper (I) include, for example, copper (I) iodide, copper (I) bromide, copper (I) iodide dimethyl sulfide, and copper (I) chloride. In some embodiments, the copper catalyst is copper (I) oxide. In other embodiments, the copper catalyst is copper (I) iodide. In other embodiments, the copper catalyst is copper (I) bromide. In yet other embodiments, the copper catalyst is copper (I) iodide dimethyl sulfide. In yet other embodiments, the copper catalyst is copper (I) chloride.

In some embodiments, compound **2** and compound **7** are reacted in the presence of base. Suitable bases include, for example, triethylamine, diisopropylethyl amine, sodium carbonate, cesium carbonate, and potassium carbonate. In some embodiments, the base is triethylamine. In other embodiments, the base is diisopropylethyl amine. In yet other embodiments, the base is sodium carbonate. In yet other embodiments, the base is cesium carbonate. In yet other embodiments, the base is potassium carbonate.

In some embodiments, compound **2** and compound **7** are reacted in the presence of solvent. Suitable solvents include, for example, terahydrofuran, dimethylformamide, dimethoxyethane, N-methylpyrrolidone, dimethylacetamide, and toluene. In some embodiments, the solvent is terahydrofuran. In other embodiments, the solvent is dimethylformamide. In yet other embodiments, the solvent is dimethoxyethane. In yet other embodiments, the solvent is N-methylpyrrolidone. In yet other embodiments, the solvent is dimethylacetamide. In further embodiments, the solvent is toluene.

In some embodiments, compound **2** and compound **7** are reacted in inert atmosphere. In some such embodiments, the inert atmosphere is provided by nitrogen. In some other such embodiments, the inert atmosphere is provided by argon.

In some embodiments, compound **2** and compound **7** are reacted at a temperature of from about 20 °C to about 130 °C. In some such embodiments, the temperature is from about 20 °C to about 50 °C. In some other such embodiments, the temperature is from about 20 °C to about 30 °C. In yet other such embodiments, the temperature is from about 40 °C to about 50 °C.

In some embodiments, compound **2** is compound **2a.** In some other embodiments, compound **2** is compound **2b.** In yet other embodiments, compound **2** is compound **2c.** In yet other embodiments, compound **2** is compound **2d.** In some such embodiments, aryl is phenyl in compound **2d.** In some other such embodiments, aryl is naphthyl in compound **2d.** In yet other embodiments, compound **2** is compound **2e.** In some such embodiments, perfluoroalkyl is perfluoro-C₁-C₆-alkyl in compound **2e.**

In some embodiments, the TMS group is removed by reacting compound **9** with base. Suitable bases, include, for example, tribasic potassium phosphate, potassium carbonate, potassium methoxide, potassium hydroxide, sodium carbonate, and sodium methoxide. In some embodiments, the base is tribasic potassium phosphate. In other embodiments, the base is potassium carbonate. In yet other embodiments, the base is potassium methoxide. In further embodiments, the base is potassium hydroxide. In yet other such embodiments, the base is sodium carbonate. In further embodiments, the base is sodium methoxide.

In some embodiments, the TMS group is removed by reacting compound **9** with fluoride source. Suitable fluoride sources include, for example, potassium fluoride, tetrabutylammonium fluoride, pyridinium fluoride, and triethylammonium fluoride. In some embodiments, the fluoride source is potassium fluoride. In other embodiments, the fluoride source is tetrabutylammonium fluoride. In yet other embodiments, the fluoride source is pyridinium fluoride. In further embodiments, the fluoride source is triethylammonium fluoride.

In some embodiments, TMS group removal is conducted in the presence of solvent. Suitable solvents include, for example, terahydrofuran, dimethylformamide, dimethoxyethane, N-methylpyrrolidone, methanol, ethanol, and isopropanol. In some embodiments, the solvent is terahydrofuran. In other embodiments, the solvent is dimethylformamide. In other embodiments, the solvent is dimethoxyethane. In yet other embodiments, the solvent is N-methylpyrrolidone. In yet other embodiments, the solvent is methanol. In yet other embodiments, the solvent is ethanol. In further embodiments, the solvent is isopropanol.

### K. N-(4-ethynylphenyl)methanesulfonamide (compound 5).

This invention also is directed, in part, to N-(4-ethynylphenyl)methanesulfonamide (compound **5**) or a salt thereof:

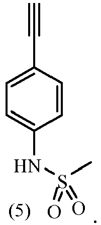

### L. Compositions.

(E)-N-(4-(3-tert-butyl-5-(2,4-dioxo-3,4-dihydropyrimidin-1(2H)-yl)-2-methoxystyryl)phenyl) methanesulfonamide (compound **I**) and its salts prepared by the above processes can be used to prepare compositions. These compositions typically also comprise one or more conventional pharmaceutically acceptable carriers, adjuvants, and/or vehicles (together referred to as "excipients").

Compositions for oral administration, and solid dosage forms in particular, are preferred. Such solid dosage forms include, for example, capsules, tablets, pills, powders, and granules. In such solid dosage forms, the compounds or salts are ordinarily combined with one or more excipients. If administered per os, the compounds or salts can be mixed with, for example, lactose, sucrose, starch powder, cellulose esters of alkanoic acids, cellulose alkyl esters, talc, stearic acid, magnesium stearate, magnesium oxide, sodium and calcium salts of phosphoric and sulfuric acids, gelatin, acacia gum, sodium alginate, polyvinylpyrrolidone, and/or polyvinyl alcohol, and then tableted or encapsulated for convenient administration. Such capsules or tablets can contain a controlled-release formulation, as can be provided in, for example, a dispersion of the compound or salt in hydroxypropylmethyl cellulose. In the case of capsules, tablets, and pills, the dosage forms also can comprise buffering agents, such as sodium citrate, or magnesium or calcium carbonate or bicarbonate. Tablets and pills additionally can be prepared with enteric coatings.

The preferred total daily dose of a compound or salt (administered in single or divided doses) is typically from about 0.001 to about 100mg/kg, more preferably from about 0.001 to about 30mg/kg, and even more preferably from about 0.01 to about 10mg/kg (*i.e.,* mg of the compound or salt per kg body weight). Dosage unit compositions can contain such amounts or submultiples thereof to make up the daily dose. In many instances, the administration of the compound or salt will be repeated a plurality of times. Multiple doses per day typically may be used to increase the total daily dose, if desired.

Factors affecting the preferred dosage regimen include the type, age, weight, sex, diet, and condition of the patient; the severity of the pathological condition; the severity of the pathological condition; pharmacological considerations, such as the activity, efficacy, pharmacokinetic, and toxicology profiles of the particular compound or salt used; whether a drug delivery system is utilized; and the specific drug combination. Thus, the dosage regimen actually employed can vary widely, and therefore, can derive from the preferred dosage regimen set forth above.

### M. Methods of Use.

This invention also is directed, in part, to a method for inhibiting replication of an RNA virus. The method comprises exposing the virus to a composition of the invention. In some embodiments, replication of the RNA virus is inhibited *in vitro.* Typically, replication of the RNA virus is inhibited *in vivo.* In some embodiments, the RNA virus whose replication is being inhibited is a single-stranded, positive sense RNA virus. In some such embodiments, the RNA virus whose replication is being inhibited is a virus from the *Flaviviridae* family. In some such embodiments, the RNA virus whose replication is being inhibited is HCV.

This invention also is directed, in part, to a method for inhibiting HCV RNA polymerase. The method comprises exposing the polymerase with a compound, salt, and/or composition of the invention. In some embodiments, HCV RNA polymerase activity is inhibited *in vitro.* Typically, HCV RNA polymerase activity is inhibited *in vivo.*

The term "inhibiting" means reducing the level of RNA virus replication/HCV polymerase activity either *in vitro* or *in vivo.* For example, if a composition of the invention reduces the level of RNA virus replication by at least about 10% compared to the level of RNA virus replication before the virus was exposed to the composition, then the composition inhibits RNA virus replication. In some embodiments, the composition can inhibit RNA virus replication by at least about 20%, at least about 30%, at least about 40%, at least about 50%, at least about 60%, at least about 70%, at least about 80%, at least about 90%, or at least about 95%.

This invention also is directed, in part, to a method for treating a disease that can be treated by inhibiting HCV RNA polymerase. Thus, this invention also is directed, in part, to a method for treating hepatitis C in an animal in need of such treatment. These methods comprise administering to the animal a compound, salt, and/or composition of the invention. In some embodiments, a therapeutically effective amount of the compound (or salt thereof) is administered to the animal. "Treating" means ameliorating, suppressing, eradicating, preventing, reducing the risk of, and/or delaying the onset of the disease being treated. Applicants specifically intend that the term "treating" encompass administration of the compositions of the invention to an HCV-negative patient that is a candidate for an organ transplant. The methods of treatment are particularly suitable for use with humans, but may be used with other animals, particularly mammals. A "therapeutically-effective amount" or "effective amount" is an amount that will achieve the goal of treating the targeted condition.

In some embodiments, the methods comprise combination therapy, wherein a compound, salt, and/or composition of the invention is co-administered with one or more additional therapeutic agents, such as, for example, another therapeutic agent used to treat hepatitis C *(e.g.,* interferon or interferon/ribavirin combination, or an HCV inhibitor such as, for example, an HCV polymerase inhibitor or an HCV protease inhibitor). The compound, salts, and/or compositions of this invention can also be co-administered with therapeutic agents other than therapeutic agents used to treat hepatitis C *(e.g.,* anti-HIV agents). In these co-administration embodiments, the compound, salts, and/or compositions of the invention and the additional therapeutic agent(s) may be administered in a substantially simultaneous manner (e.g., or within about 5 min of each other), in a sequential manner, or both. It is contemplated that such combination therapies may include administering one therapeutic agent multiple times between the administrations of the other. The time period between the administration of each agent may range from a few seconds (or less) to several hours or days, and will depend on, for example, the properties of each composition and active ingredient (e.g., potency, solubility, bioavailability, half-life, and kinetic profile), as well as the condition of the patient.

This invention also is directed, in part, to a use of the compound, salts, and/or compositions of the invention, and, optionally one or more additional therapeutic agents to prepare a medicament.

In some embodiments, the medicament is for co-administration with one or more additional therapeutic agents.

In some embodiments, the medicament is for inhibiting replication of an RNA virus.

In some embodiments, the medicament is for treating hepatitis C.

This invention also is directed, in part, to one or more compositions of the invention, and, optionally one or more additional therapeutic agents, for use as a medicament. In some embodiments, the medicament is for inhibiting replication of an RNA virus. In other embodiments, the medicament is for treating hepatitis C.

### EXAMPLES

The following examples are merely illustrative, and not limiting to this disclosure in any way.

### Example 1. Preparation of 1-(3-tert-Butyl-5-iodo-4-methoxyphenyl)pyrimidine-2,4(1H,3H)-dione.

### Part A. Preparation of 2-tert-butyl-4,6-diiodophenol.

2-*tert*-Butylphenol (99.95g, 665.36mmol) was dissolved in 1250mL methanol and converted to the corresponding phenoxide with 31.96g (799.0mmol, 1.2equiv.) of sodium hydroxide by stirring the sodium hydroxide pellets at room temperature, and then cooling the reaction mixture in an ice/salt bath. Sodium iodide (299.34g, 1997.07mmol, 3.0equiv.) and 8.3% bleach (1265.83g, 1411.39mmol, 2.1equiv.) were added to the cold reaction solution in four equal portions, the bleach being added while keeping the reaction mixture at <0°C. 500mL of 20% (w/w) sodium thiosulfate solution was added over an 18-minute period, with the temperature rising from -0.6°C to 2.5°C. The pH of the reaction mixture was adjusted to approximately 3 by adding 197.5mL of conc. HCl over a period of 97min with the reaction temperature going from 1.2°C to 4.1°C. The resulting slurry was filtered, and the wet cake washed with ~ 2L of water. The wet cake was left on the Buchner funnel under vacuum overnight (approximately 15h) to yield 289.33g (potency adjusted yield = 254.61g) of the title product.

### Part B. Preparation of 1-tert-butyl-3,5-diiodo-2-methoxybenzene.

The product from **Part A** (93%assay, 21.6g, 50mmol) was dissolved in 140mL of acetone. Methyl iodide (4.2mL, 67.5mmol, 1.35equiv.) was added, followed by 50% aqueous sodium hydroxide (5.0g, 62.5mmol, 1.25equiv.). The reaction was stirred overnight, then concentrated to approximately 50-60mL. 80mL of heptanes was added followed by 50mL of water, and the layers were shaken and separated, and the aqueous layer was back extracted with 20mL of heptanes. The organic layers were combined and washed twice with 50mL each of 10% aqueous NaCl to afford 91.1 grams of a heptane solution, which assayed to 19.1g of the title compound.

### Part C. Preparation of 1-(3-tert-Butyl-5-iodo-4-methoxyphenyl)pyrimidine-2,4(1H,3H)-dione.

Uracil (33.3g, 297mmol, 1.2equiv.), K₃PO₄ (106g, 500mmol, 2.1equiv.), CuI (4.6g, 24.2mmol, 0.1equiv.), and *N*-(2-cyanophenyl)picolinamide (6.4g , 28.7mmol, 0.12equi.) were charged to a flask and inerted with argon. The 1-*tert*-butyl-3,5-diiodo-2-methoxybenzene was solvent switched into MeCN, dissolved in 1L DMSO and sparged with argon and added to the solids. The reaction was heated to 60°C for 16h. After cooling, the reaction was diluted with 2L EtOAc and washed with 2.6L water (back extracted with 3 x 1L EtOAc). The combined organic layers were washed with 2 x 1L of 0.25M (CuOAc)₂ then 2 x 830mL 15% NH₄Cl then 800mL brine. The organic layer was then concentrated and chased with 1L heptane, then triturated with refluxing 85:15 (v/v) heptane:iPrOAc for 4h. After cooling, the product was collected by filtration and washed with an additional 330mL of 85:15 v/v heptanes:EtOAc to yield after drying 66.9g (70% yield) of the product as a white solid.

### Example 2. Preparation of 1-(3-tert-butyl-4-methoxy-5-((trimethylsilyl)ethynyl)phenyl) pyrimidine-2,4(1H,3H)-dione.

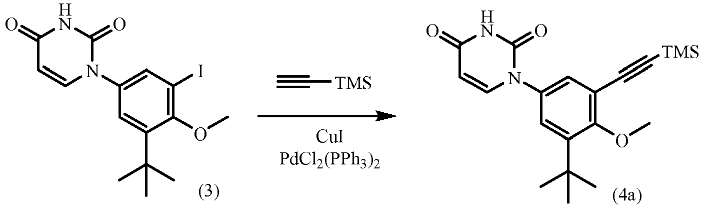

To a flask with condenser, was successively charged 1-(3-tert-butyl-5-iodo-4-methoxyphenyl)pyrimidine-2,4(1H,3H)-dione (**3**) (30 g; 75 mmol), copper(I) iodide (0.086 g; 0.450 mmol) and bis(triphenylphosphine)palladium(II) chloride (0.158 g; 0.225 mmol) then charge a solution previously purged with nitrogen of tetrahydrofuran (60 ml) and triethylamine (55.4 ml; 397 mmol). The mixture was stirred at 25°C for 15 minutes. To the solution was added dropwise ethynyltrimethylsilane (8.83 g; 90 mmol) and the solution mixed at 25°C for 15 minutes before adjusting the temperature to 45°C for 18.5 hours. The reaction mixture cooled to ambient and diluted with ethyl acetate (230mL). The reaction mixture was washed twice with 1% *l*-Cysteine / 5% bicarbonate solution followed by 5% sodium bicarbonate and then a 15% sodium chloride solution. The organic layer was treated with activated carbon and concentrated under reduced pressure. The residual ethyl acetate was chased by charging heptane and concentrating under reduced pressure to an appropriate volume. The heptane slurry was heated at reflux for 2 hours then cooled to 25°C for 12 hours. The solids were filtered and washed with heptane and dried by vacuum filtration to give 25 grams of the titled compound **4a.** ¹H NMR (400 MHz, CDCl₃) δ 8.8 (s, 1H), 7.34 - 7.27 (m, 2H), 7.21 (d, *J* = 2.8, 1H), 5.83 (d, *J* = 7.9, 1H), 4.13 (s, 3H), 1.41 (s, 9H), 0.29 (s, 9H). MS (APCI) *m*/*z* 371.2 (M+H)+.

### Example 3. Preparation of 1-(3-tert-butyl-5-ethynyl-4-methoxyphenyl)pyrimidine-2,4 (1H,3H)-dione.

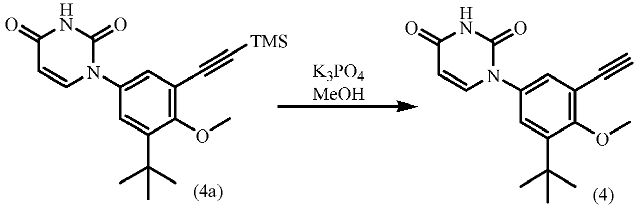

To a vessel was charged Example **4a** (11 g; 29.7 mmol) and Methanol (60 ml). To the mixture was added a 19% potassium phosphate tribasic aqueous solution (30 ml) then the temperature was adjusted at 45°C and mixed for 3 hours. The reaction temperature was cooled at 35°C and a 13% acetic acid solution (69 ml) was added to the mixture drop-wise. The slurry was mixed at 55 °C for 90 minutes cooled to 25 °C, and stirred for 12 hours. The solids were filtered, washed with water, and dried to give 8.78 grams titled compound **4.** ¹H NMR (400 MHz, CDCl₃) δ 8.77 (bs, 1H), 7.30 (d, *J* = 2.7, 1H),7.27 (d, *J*=7.9), 7.21 (d, *J =* 2.7, 1H), 5.80 (d, *J =* 7.9, 1H), 4.09 (s, 3H), 3.41 (s, 1H), 1.38 (s, 9H). MS (APCI) *m*/*z* 299.2(M+H)+.

### Example 4. Preparation of N-(4-((3-tert-butyl-5-(2,4-dioxo-3,4-dihydropyrimidin-1(2H)-yl)-2-methoxyphenyl) ethynyl)phenyl)methanesulfonamide.

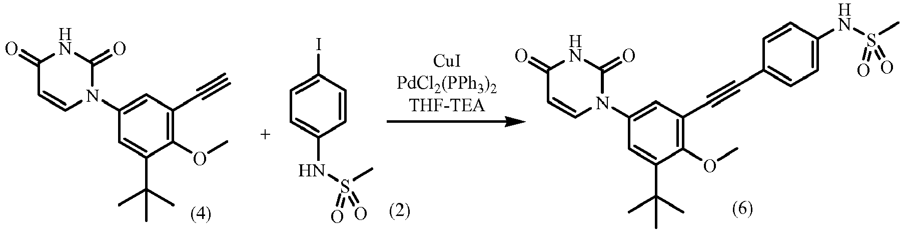

To a flask was added 1-(3-tert-butyl-5-ethynyl-4-methoxyphenyl)pyrimidine-2,4(1H,3H)-dione (**4**) (6.0 g; 20.11 mmol) and N-(4-iodophenyl)methanesulfonamide (**2**) (6.0 g; 20.11 mmol). To this was added bis(triphenylphosphine)palladium(II) chloride (0.141 g; 0.201 mmol) and copper (I) chloride (0.077 g; 0.402 mmol). To this was charged a solution tetrahydrofuran (60 ml) and triethylamine (17.8 ml; 121 mmol) previously purged with nitrogen. The reaction was stirred overnight at 25°C.

Isolation of the solids: The reaction mixture was solvent exchanged into methanol and the resulting slurry filtered. The solids were washed with methanol and then reslurried in approximately 7.5 mL methanol per gram solid. This was heated to reflux, cooled to ambient, filtered, and washed with methanol to give the title compound **6**. 1H NMR (400 MHz, DMSO) δ 11.40 (bs, 1H), 10.08 (bs, 1H), 7.71 (d, *J* = 7.8, 1H), 7.57 - 7.51 (m, 2H), 7.45 (d, *J* = 2.6, 1H), 7.31-7.21 (m, 3H), 5.63 (d, *J* = 7.8, 1H), 4.08 (s, 3H), 3.05 (s, 3H), 1.35 (s, 9H). MS (APCI) *m*/*z* 468.2 (M+H)+⁻

### Example 5. Preparation of (E)-N-(4-(3-tert-butyl-5-(2,4-dioxo-3,4-dihydropyrimidin-1(2H)-yl)-2-methoxystyryl)phenyl)methanesulfonamide.

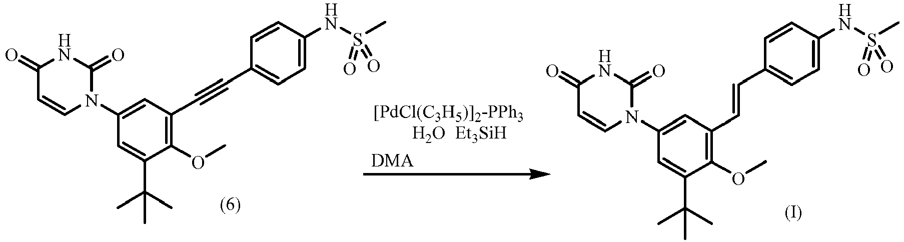

To a flask was successively added N-(4-((3-tert-butyl-5-(2,4-dioxo-3,4-dihydropyrimidin-1(2H)-yl)-2-methoxyphenyl)ethynyl)phenyl)methanesulfonamide (**6**) (20 g; 42.60 mmol); allylpalladium(II) chloride (0.39 g; 1.06 mmol); triphenylphosphine (1.12 g; 4.26 mmol) and a solution of dimethylacetamide (80 ml) / water (2.3 ml) previously purge with nitrogen. The solution was heated at 70 °C for 30 minutes. To the reaction mixture was then added over 3 hours triethylsilane (7.42 g; 63.90 mmol). The mixture was stirred for 12 hours at 70 °C. The reaction was cooled at 23 °C then diluted with tetrahydrofuran (180 ml) and successively washed with a solution of 1% Cysteine / 2.5% NaHCO₃ / 7% NaCl and a solution of 2.5% NaHCO₃ / 7% NaCl. The organic layer was treated with activated carbon and the filtrate was concentrated down and solvent switched to methanol. The slurry was mixed at 62 °C for 60 minutes, cooled down to 25 °C, mixed for 2 hours, filtered, and washed with methanol to give the titled compound. ¹H NMR (400 MHz, DMSO-D6) δ ppm 1.35 (s, 9 H) 2.99 (s, 3 H) 3.76 (s, 3 H) 5.62 (d, J=7.82 Hz, 1 H) 7.12 - 7.29 (m, 5 H) 7.51 - 7.65 (m, 3 H) 7.72 (d, J=7.82 Hz, 1 H) 9.83 (s, 1 H) 11.39 (s, 1 H) MS (APCI) *m*/*z* 470.1(M+H)+.

### Example 6. Preparation of (E)-N-(4-(3-tert-butyl-5-(2,4-dioxo-3,4-dihydropyrimidin-1(2H)-yl)-2-methoxystyryl)phenyl)methanesulfonamide.

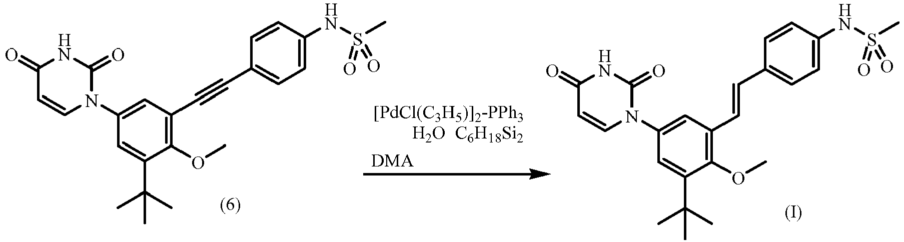

To a flask was added allylpalladium(II) chloride (0.98 g; 2.67 mmol) and triphenyl phosphine (2.8 g; 10.69 mmol). Vacuum and nitrogen purged. Sparged dimethylacetamide (50 ml) (nitrogen bubbled) was added and the solution was mixed at room temperature for 2hrs. N-(4-((3-tert-butyl-5-(2,4-dioxo-3,4-dihydropyrimidin-1(2H)-yl)-2-methoxyphenyl)ethynyl)phenyl)methanesulfonamide (**6**) (50 g; 107 mmol) was dissolved in 100mL of nitrogen sparged dimethylacetamide in a separate flask. This was inerted with nitrogen after the dissolution. The catalyst solution was transferred to the second flask containing substrate (6). Water (5 ml) and 1,1,1,2,2,2-hexamethyldisilane (16.1 mL; 160 mmol) were then added to the reaction mixture. The mixture was heated to 81 °C for total of 18 hours. The reaction mixture was cooled to ambient temperature and quenched with 500 ml THF and 500 ml of a solution of 7% NaCl, 2.5% NaHCO₃ and 1% of cysteine. The quenched mixture was stirred for 1 hour and filtered to remove particulates. The filtrate was separated. The aqueous layer was back extracted with 250mL THF and the combined organic layers washed again with 250 ml of a solution of solution of 7% NaCl, 2.5% NaHCO₃ and 1% of cysteine. The organics were treated with activated carbon and the filtrate concentrated and solvent switched to 500 ml of methanol. The slurry was mixed at 50 °C for 60 minutes, cooled to ambient overnight. The slurry was filtered, and the solids washed with methanol, and dried to give 43.86 grams of the titled compound. ¹H NMR (400 MHz, DMSO-D6) δ ppm 1.35 (s, 9 H) 2.99 (s, 3 H) 3.76 (s, 3 H) 5.62 (d, J=7.82 Hz, 1 H) 7.12 - 7.29 (m, 5 H) 7.51 - 7.65 (m, 3 H) 7.72 (d, J=7.82 Hz, 1 H) 9.83 (s, 1 H) 11.39 (s, 1 H) MS (APCI) *m*/*z* 470.1 (M+H)+.

### Example 7. Preparation of (E)-N-(4-(3-tert-butyl-5-(2,4-dioxo-3,4-dihydropyrimidin-1(2H)-yl)-2-methoxystyryl)phenyl)methanesulfonamide.

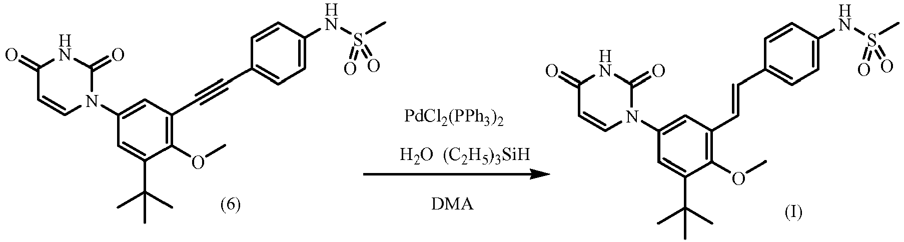

To a vessel N-(4-((3-tert-butyl-5-(2,4-dioxo-3,4-dihydropyrimidin-1(2H)-yl)-2-methoxyphenyl)ethynyl)phenyl)methanesulfonamide (6) (5.00 g; 10.69 mmol) and bis(triphenyl phosphine)palladium(II) chloride (0.375 g; 5.35 mmol) are charged and the vessel is purged with nitrogen. Nitrogen purged dimethylacetamide (20 ml) and water (0.578 g, 32.1 mmol) are charged to the vessel and the mixture was heated to 70°C. Triethylsilane (2.49 g, 21.4 mmol) was charged over 2 hours at a temperature of 70°C. The mixture was heated at 70°C for approximately 5 hours and cooled to ambient temperature. The mixture was diluted with tetrahydrofuran (40 g) and extracted with a 1% Cysteine / 2.5% NaHCO₃ / 7% NaCl solution. The solids which formed were removed by filtration and washed with tetrahydrofuran (18 g). The aqueous layer was extracted with tetrahydrofuran (18 g). The combined tetrahydrofuran extracts were washed successively with a 1% Cysteine / 2.5% NaHCO₃ / 7% NaCl solution and a 2.5% NaHCO₃ / 7% NaCl solution. Methanol (28 g) was charged and the solution concentrated to 28 g. Methanol (28 g) was charged and the product was allowed to crystallize. Methanol (28 g) was charged and the slurry was concentrated to 45 g. Methanol (60 g) was charged and the mixture concentrated to 75 g. The slurry was heated to 60°C for 30 minutes and then cooled to ambient temperature. The solids were isolated by filtration, washed with methanol (2 x 8 gm) and vacuum dried at 50°C to provide 4.42 g of the titled compound. ¹H NMR (400 MHz, DMSO-D6) δ ppm 1.35 (s, 9 H) 2.99 (s, 3 H) 3.76 (s, 3 H) 5.62 (d, J=7.82 Hz, 1 H) 7.12 - 7.29 (m, 5 H) 7.51 - 7.65 (m, 3 H) 7.72 (d, J=7.82 Hz, 1 H) 9.83 (s, 1 H) 11.39 (s, 1 H). MS (APCI) *m*/*e* 470.1 (M+H)+.

### Example 8. Genotoxic Testing.

As discussed above, compound I (including salts thereof) is useful as a drug for treating HCV in humans. Thus, it is important that it be so prepared that it is safe for administration to humans. As one skilled in the art would understand, one aspect of drug safety is the minimization of the amount of genotoxic impurities that are associated with the production of a given drug, and, can therefore also be present in the drug product. A couple of well established tests are used to measure the genotoxicity of chemical compounds. One such test is the computer-based Derek analysis, and the other - the Ames test, which is a biological assay that assesses the mutagenic potential of chemical compounds. As can be seen from the results below, the process for preparing compound I (or salt thereof) of this invention (shown in Schemes 3 and 4 below) results in the use and/or formation of fewer genotoxic impurities than the process shown in Schemes 1 and 2 below.

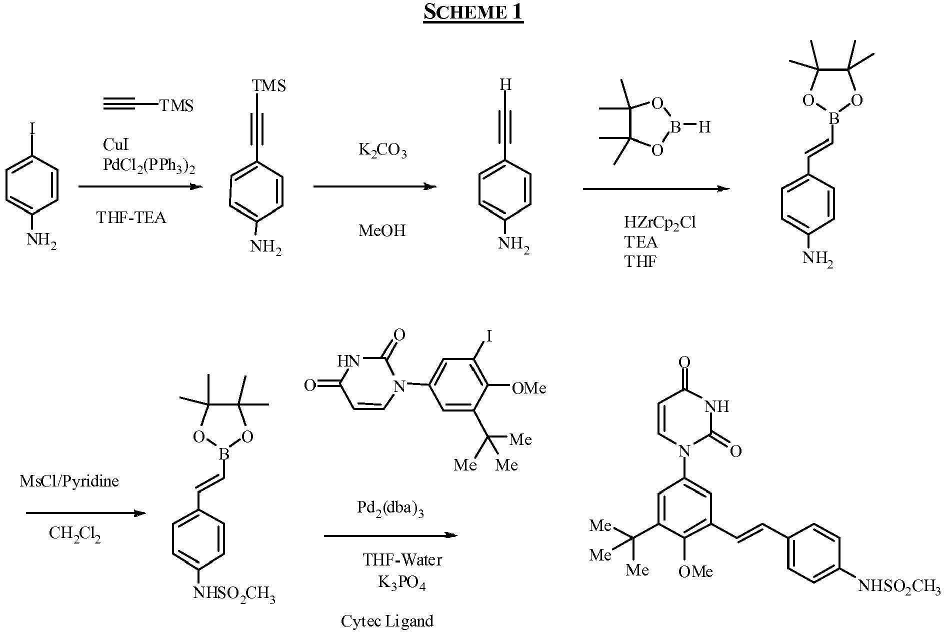

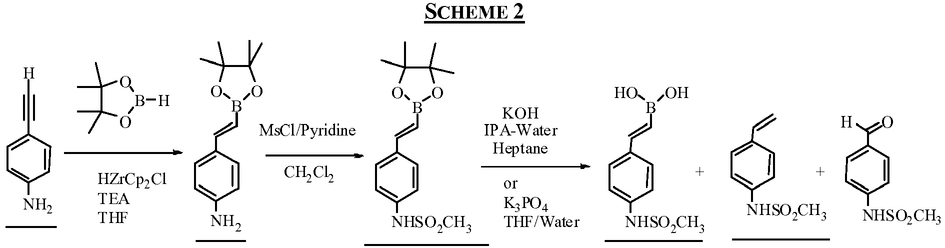

The five underlined compounds in Scheme 2 are genotoxic based on Derek analysis and Ames test.

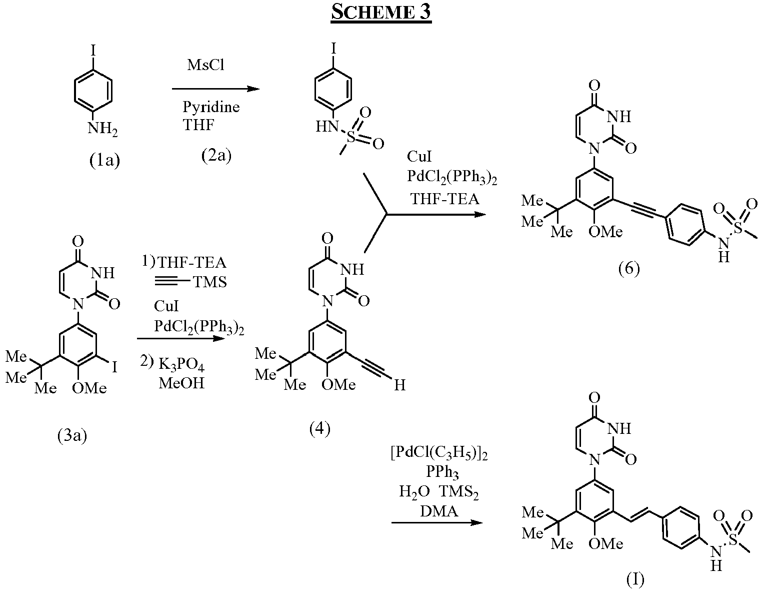

Compounds 1a and 2a in Scheme 2 tested negative in Ames test. Compounds 3a, 4, and 6 tested safe in Derek analysis and thus no Ames tests were performed.

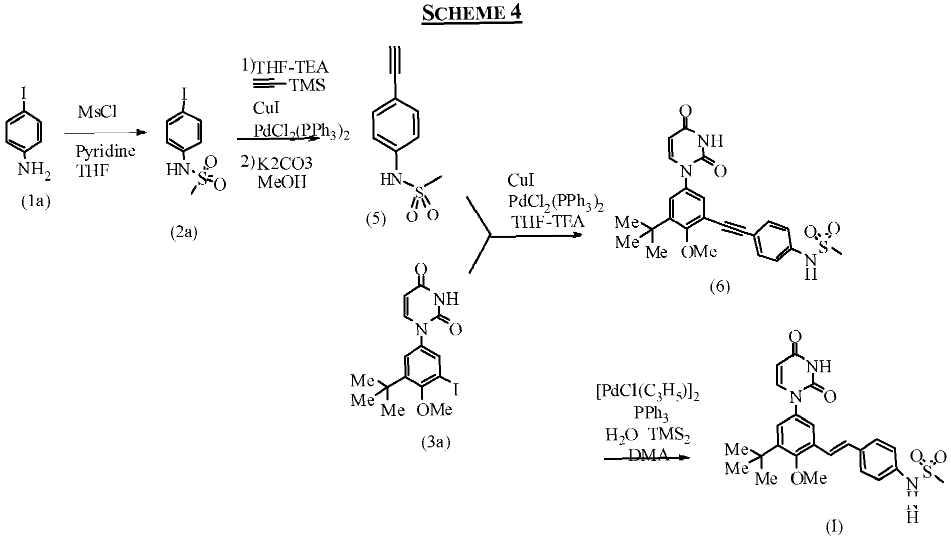

## Claims

1. A process for preparing (E)-N-(4-(3-tert-butyl-5-(2,4-dioxo-3,4-dihydropyrimidin-1(2H)-yl)-2-methoxystyryl)phenyl)methanesulfonamide (compound **I**) or a salt thereof, wherein the process comprises:
reducing N-(4-((3-tert-butyl-5-(2,4-dioxo-3,4-dihydropyrimidin-1(2H)-yl)-2-methoxyphenyl) ethynyl)phenyl)methanesulfonamide (compound **6**):

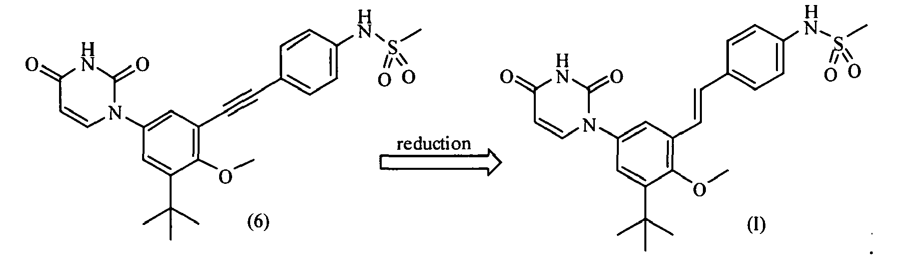

2. The process of claim **1,** wherein compound **6** is reduced using silane.

3. The process of claim **2,** wherein the silane is selected from the group consisting of triethylsilane, phenylsilane, diphenylsilane, tripropylsilane, triphenylsilane, tribenzylsilane, 1,1,1,3,5,5,5-heptamethyltrisiloxane, tributylsilane, di-tert-butylsilane, diethyloxysilane, dimethoxysilane, phenyldiethoxyethylsilane, dimethylsilane, halosilane, and tris(trimethylsilyl)silane.

4. The process of claim **1,** wherein compound 6 is reduced using disilane.

5. The process of claim **4,** wherein the disilane is selected from the group consisting of hexamethyldisilane, hexaphenyldisilane, 1,2-diphenyltetramethyldisilane, 1,2-dimethyl-1,1,2,2,-tetraphenyldisilane, 1,1,2,2-tetramethyldisilane, 1,2-diethoxy-1,1,2,2-tetramethyldisilane, 1,2-dimethoxy-1,1,2,2-tetramethyldisilane, and hexamethoxydisilane.

6. The process of any one of claims **1-5,** wherein compound **6** is reduced in the presence of catalyst.

7. The process of claim **6,** wherein the catalyst is palladium catalyst.

8. The process of claim **7,** wherein the catalyst is selected from the group consisting of tetrakis(triphenylphosphine)palladium (0), dichlorobis(tri-o-tolylphosphine)palladium (II), palladium (II) acetate, [1,1'-bis(diphenylphosphino)ferrocene]dichloropalladium (II), tris(dibenzylideneacetone) dipalladium (0), dichloro(dibenzylideneacetone)dipalladium (II), dichlorotris(cyclohexylphosphine)- palladium (II), dichlorobis(triphenylphosphine) palladium (II), and chloro(η3-allyl)palladium(II) dimer-triphenylphosphine.

9. The process of any one of claims **1-8,** wherein compound **6** is reduced in the presence of solvent.

10. The process of claim **9,** wherein the solvent is selected from the group consisting of terahydrofuran, dimethylformamide, dimethylacetamide, and N-methylpyrrolidone.

11. The process of any one of claims **1-10,** wherein compound **6** is prepared by reacting 1-(3-tert-butyl-5-ethynyl-4-methoxyphenyl)pyrimidine-2,4(1H,3H)-dione (compound **4**) with compound **2** selected from the group consisting of N-(4-iodophenyl)methanesulfonamide (compound **2a**), N-(4-bromophenyl)methanesulfonamide (compound **2b**), N-(4-chlorophenyl)methanesulfonamide (compound **2c**), N-(4-[(arylsulfonyl)oxy]phenyl)methanesulfonamide (compound **2d**), and N-(4-[(perfluoroalkylsulfonyl)oxy]phenyl)methanesulfonamide (compound **2e**):

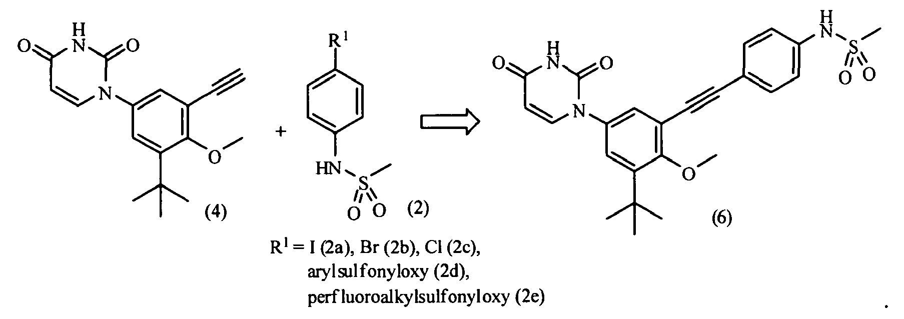

12. The process of claim **11,** wherein compound **4** and compound **2** are reacted in the presence of base.

13. The process of any one of claims **11** and **12,** wherein compound **4** and compound 2 are reacted in the presence of solvent.

14. The process of any one of claims **11-13,** wherein compound **4** is prepared by:
reacting ethynyltrimethylsilane (≡TMS) (compound **7)** with compound **3** selected from the group consisting of 1-(3-tert-butyl-5-iodo-4-methoxyphenyl) pyrimidine-2,4(1H,3H)-dione (compound **3a),** 1-(3-bromo-5-tert-butyl-4-methoxyphenyl)pyrimidine-2,4(1H,3H)-dione (compound **3b**), and 1-(3-tert-butyl-5-chloro-4-methoxyphenyl)pyrimidine-2,4(1H,3H)-dione (compound **3c**) thus forming 1-(3-tert-butyl-4-methoxy-5-((trimethylsilyl)ethynyl)phenyl)pyrimidine-2,4(1H,3H)-dione (compound **8**): 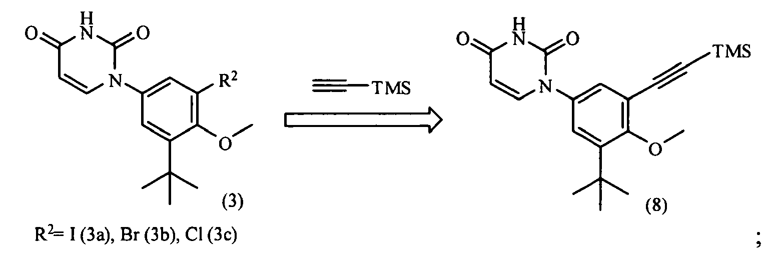 and
removing the trimethylsilyl (TMS) group from the formed 1-(3-tert-butyl-4-methoxy-5-((trimethylsilyl)ethynyl)phenyl)pyrimidine-2,4(1H,3H)-dione (compound **8**):

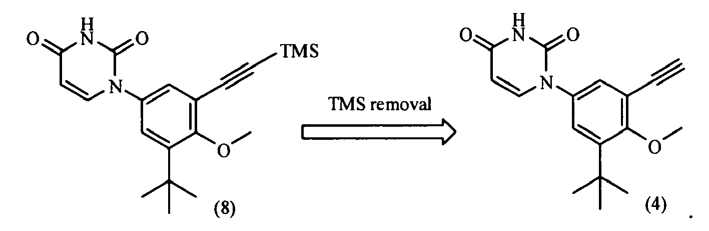

15. The process of any one of claims **1-10,** wherein compound **6** is prepared by reacting N-(4-ethynylphenyl)methanesulfonamide (compound **5**) with compound **3** selected from the group consisting of 1-(3-tert-butyl-5-iodo-4-methoxyphenyl)pyrimidine-2,4(1H,3H)-dione (compound **3a**), 1-(3-bromo-5-tert-butyl-4-methoxyphenyl)pyrimidine-2,4(1H,3H)-dione (compound **3b**), and 1-(3-tert-butyl-5-chloro-4-methoxyphenyl)pyrimidine-2,4(1H,3H)-dione (compound **3c**) thus forming N-(4-((3-tert-butyl-5-(2,4-dioxo-3,4-dihydropyrimidin-1(2H)-yl)-2-methoxyphenyl) ethynyl)phenyl) methanesulfonamide (compound **6**):

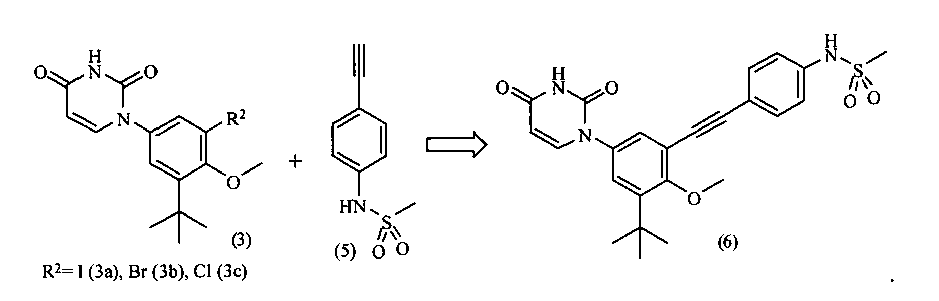

16. The process of claim **15,** wherein compound **5** is prepared by:
reacting ethynyltrimethylsilane (≡TMS) (compound **7**) with compound **2** selected from the group consisting of N-(4-iodophenyl)methanesulfonamide (compound **2a**), N-(4-bromophenyl) methanesulfonamide (compound **2b**), N-(4-chlorophenyl)methanesulfonamide (compound **2c**), N-(4-[(arylsulfonyl)oxy]phenyl)methanesulfonamide (compound **2d**), and N-(4-[(perfluoroalkylsulfonyl) oxy]phenyl)methanesulfonamide (compound **2e**) thus forming N-(4-((trimethylsilyl)ethynyl)phenyl) methanesulfonamide (compound **9**): 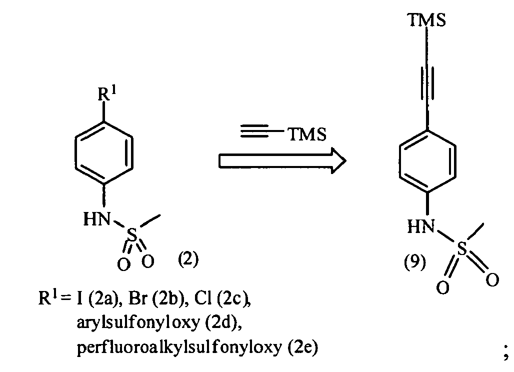 and
removing the trimethylsilyl (TMS) group from the formed N-(4-((trimethylsilyl)ethynyl) phenyl) methanesulfonamide (compound **9**):

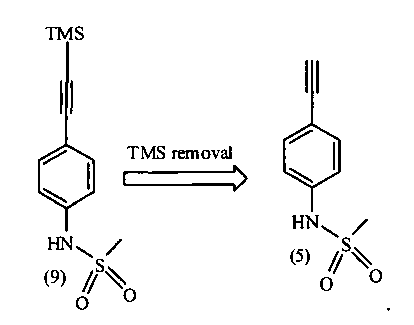

17. A compound or salt thereof, wherein the compound is selected from the group consisting of compounds corresponding in structure to the following formulae: 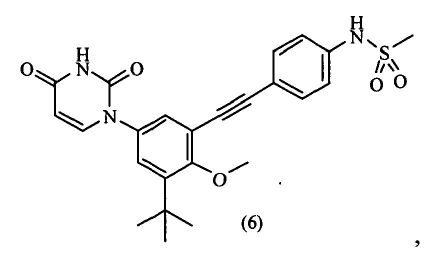 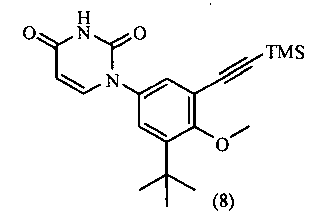 and

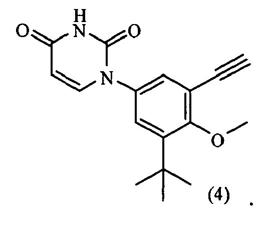

## Patentansprüche

1. Ein Prozess zur Zubereitung (E)-N-(4-(3-tert-butyl-5-(2,4-dioxo-3,4-dihydropyrimidin-1(2H)-yl)-2-methoxystyryl)phen yl)methanesulfonamide (compound I) oder ein Salz hieraus, worin der Prozess besteht aus:
verringerte N-(4-((3 -tert-butyl-5 -(2,4-dioxo-3,4-dihydropyrimidin-1 (2H)-yl)-2-methoxyphenyl)ethynyl)phenyl)methanesulfonamide (Verbund **6**):

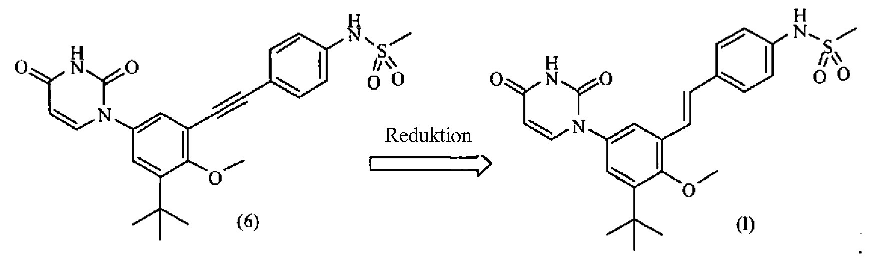

2. Der Prozess des Anspruchs **1,** worin der Verbund **6** reduziert is, verwendet Siliziumwasserstoff.

3. Der Prozess des Anspruchs **2,** worin der Siliziumwasserstoff ausgewählt ist ausgewählt aus der Gruppe bestehend aus Triethylsiliziumwasserstoff, Phenylsiliziumwasserstoff, Diphenylsiliziumwasserstoff, Tripropylsiliziumwasserstoff, Triphenylsiliziumwasserstoff, Tribenzylsiliziumwasserstoff, 1,1,1,3,5,5,5-Heptamethyltrisiloxan, Tributylsilan, di-tert-butylsilan, diethyloxysilan, dimethoxysilan, phenyldiethoxyethylsilan, dimethylsilan, halosilan und tris(trimethylsilyl)silan.

4. Der Prozess des Anspruchs **1,** worin der Verbund **6** reduziert ist und Disilan verwendet.

5. Der Prozess des Anspruchs **4,** worin Disilan ausgewählt wird aus einer Gruppe von hexamethyldisilan, hexaphenyldisilan, 1,2-diphenyltetramethyldisilan, 1,2-dimethyl-1,1,2,2,-tetraphenyldisilan, 1,1,2,2-tetramethyldisilan, 1,2-diethoxy-1,1,2,2-tetramethyldisilan, 1,2-dimethoxy-1,1,2,2-tetramethyldisilan und hexamethoxydisilan.

6. Der Prozess einer jeder der Ansprüche **1-5,** worin der Verbund **6** reduziert ist in der Präsenz des Katalysator.

7. Der Prozess des Anspruchs **6,** worin der Katalysator Palladium Katalysator ist.

8. Der Prozess des Anspruchs 7, worin der Katalysator aus der Gruppe ausgewählt ist aus tetrakis(triphenylphosphin)palladium (0), dichlorobis(tri-o-tolylphosphin)palladium (II), palladium (II) azetat, [1,r-bis(diphenylphospruno)ferrocen]dichloropalladiurn (II), tris(dibenzylideneazeton) dipalladium (0), dichloro**(diben2ylideneazeton)**dipalladium (II), dichlorotris(cyclohexylphosphin)- palladium (II), dichlorobis(triphenylphosphin) palladium (II) und chloro(η3-allyl)palladium(II) dimer-triphenylphosphin.

9. Der Prozess einer jeder der Ansprüche **1-8,** worin der Verbund **6** reduziert ist in der Präsenz der Lösung.

10. Der Prozess des Anspruchs **9,** worin die Lösung wird ausgewählt aus der Gruppe bestehend aus terahydrofuran, dimethylformamid, dimethylacetamid und N-methylpyrrolidone.

11. Der Prozess eines jeden des Anspruchs **1-10,** worin der Verbund bestehend ist aus **6** wird zubereitet durch die Reaktion 1-(3-tert-butyl-5-ethynyl-4-methoxyphenyl)pyrimidin-2,4(1H,3H)-dione (Verbund **4**) mit Verbund **2** ausgewählt aus der Gruppe aus N-(4-iodophenyl)methanesulfonamid (Verbund **2a**), N-(4-bromophenyl)methanesulfonamid (Verbund **2b**), N-(4-Chlorophenyl)methanesulfonamid (Verbund **2c**), N-(4-[(arylsulfonyl)oxy]phenyl)methanesulfonamid (Verbund **2d**) und N-(4-[(perfluoroalkylsulfonyl)oxy]phenyl)methanesulfonamid (Verbund **2e**) :

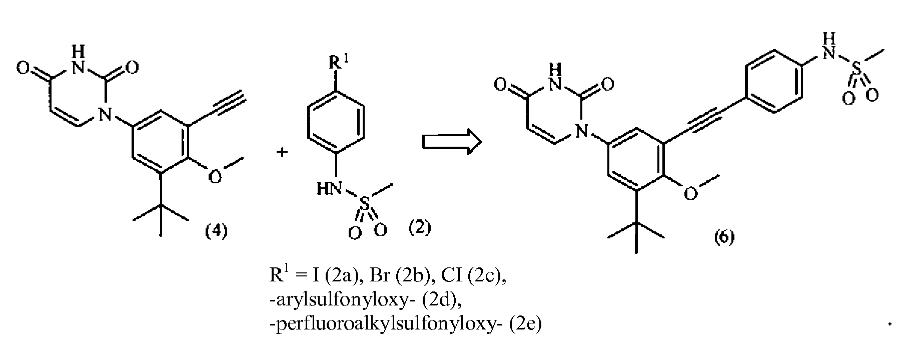

12. Der Prozess des Anspruchs **11**, worin Verbund **4** und Verbund **2** reagieren miteinander in der Präsenz der Basis.

13. Der Prozess eines jeden der Ansprüche **11** und **12,** worin der Verbund **4** und Verbund **2** reagieren miteinander in der Präsenz der Lösung.

14. Der Prozess einer jeden der Ansprüche **11-13**, worin der Verbund **4** zubereitet wird durch:
reagiert mit ethynyltrimethylsilan (≡TMS) (Verbund **7**) mit Verbund **3** ausgewählt aus der Gruppe bestehend aus 1-(3-tert-butyl-5-iod-4-methoxyphenyl) pyrimidin-2,4(1H,3H)-dion (Verbund **3a**)**,**
1-((3-brom-5-tert-butyl-4-methoxyphenyl)pyrimidin-2,4(1H,3H)-dion (Verbund **3b**) und
1-((3-tert-butyl-5-chlor-4-methoxyphenyl)pyrimidin-2,4(1H,3H)-dion (Verbund **3c**) und so formt
1-((3-tert-butyl-4-methoxy-5-((trimethylsilyl)ethynyl)phenyl)pyrimidin-2,4(1H,3H)-dion (Verbund **8**): 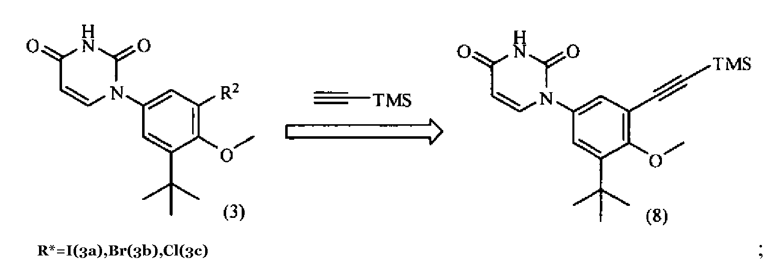 und
wobei trimethylsilyl (TMS) Gruppe von den geformten folgenden herausgelöst wird 1-(3-tert-butyl-4-methoxy-5-((trimethylsilyl)ethynyl)phenyl)pyrimidin-2,4(1H,3H)-dion (Verbund **8**):

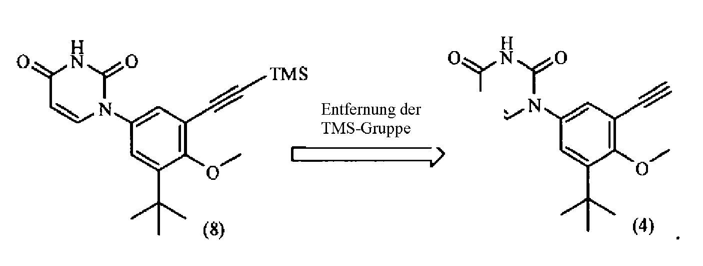

15. Der Prozess eines jeden Anspruchs **1-10,** worin der Verbund **6** zubereitet ist durch die Reaktion mit N-(4-ethynylphenyl)methanesulfonamid (Verbund **5**) mit Verbund **3** ausgewählt aus der Gruppe bestehend aus
1-((3-tert-butyl-5-iod-4-methoxyphenyl)pyrimidin-2,4(1H,3H)-dion (Verbund **3a**),
1-((3-brom-5-tert-butyl-4-methoxyphenyl)pyrimidin-2,4(1H,3H)-dion (Verbund **3b**) und
1-((3-tert-butyl-5-chlor-4-methoxyphenyl)pyrimidin-2,4(1H,3H)-dion (Verbund **3c**) in dieser Weise formen dies
N-(4-((3-tert-butyl-5-(2,4-dioxo-3,4-dihydropyrimidin-1(2H)-yl)-2-methoxyphenyl) ethynyl)phenyl) methanesulfonamid (Verbund **6**):

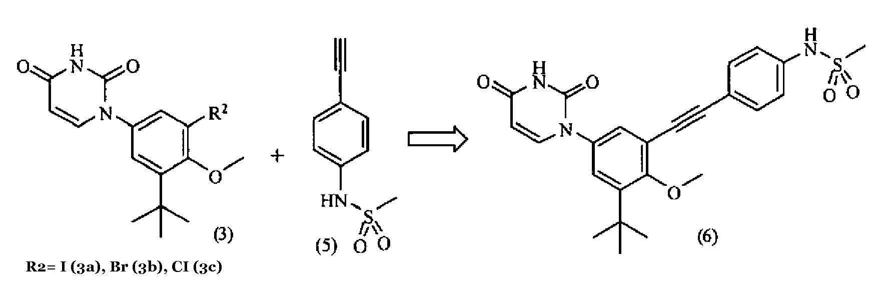

16. Der Prozess des Anspruchs **15**, worin der Verbund **5** wird zubereitet durch: Reaktion ethynyltrimethylsilan (≡TMS) (Verbund **7**) mit Verbund **2** ausgewählt aus der Gruppe bestehend aus N-(4-iodophenyl)methanesulfonamid (Verbund **2a**), N-(4-bromophenyl) methanesulfonamid (Verbund **2b**)**,** N-(4-chlorophenyl)methanesulfonamid (Verbund **2c**), N-(4-[(arylsulfonyl)oxy]phenyl)methanesulfonamid (Verbund **2d**) und N-(4-[(perfluoroalkylsulfonyl) oxy]phenyl)methanesulfonamid (Verbund **2e**) in dieser Weise formen diese N-(4-((trimethylsilyl)ethynyl)phenyl) methanesulfonamid (Verbund **9**): 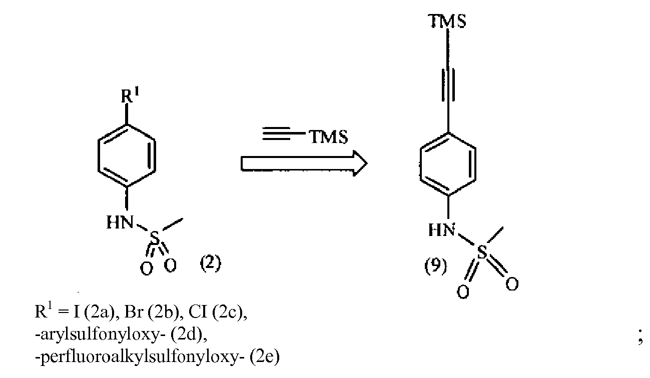 und
wobei trimethylsilyl (TMS) Gruppe aus den geforment herausgelöst wird N-(4-((trimethylsilyl)ethynyl) phenyl) methanesulfonamid (Verbund **9):**

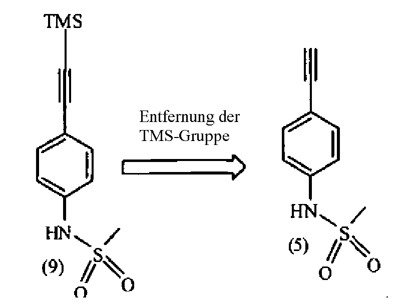

17. Ein Verbund oder Salz ist deshalb, worin der Verbund ausgewählt wird aus der Gruppe bestehend aus Verbunden, die in der Struktur der folgenden Formel korrespondieren: 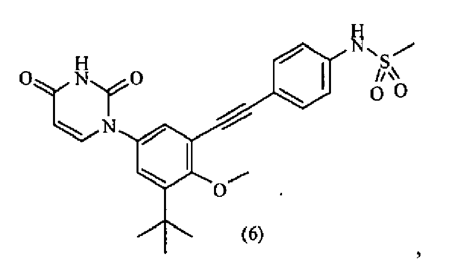 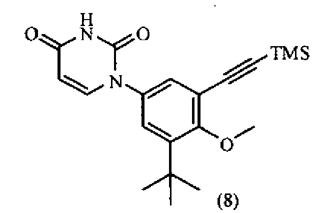 und

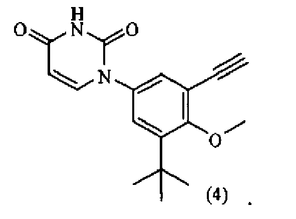

## Revendications

1. Procédé de préparation de (E)-N-(4-(3-tert-butyl-5-(2,4-dioxo-3,4-dihydropyrimidine-1(2H)-yl)-2-méthoxystyryl)phényl)méthanesulfonamide (composé **I**) ou un sel de celui-ci, dans lequel le procédé comprend :
la réduction de N-(4-((3-tert-butyl-5-(2,4-dioxo-3,4-dihydropyrimidine-1(2H)-yl)-2-méthoxyphényl)éthynyl)phényl)méthanesulfonamide (composé **6**) :

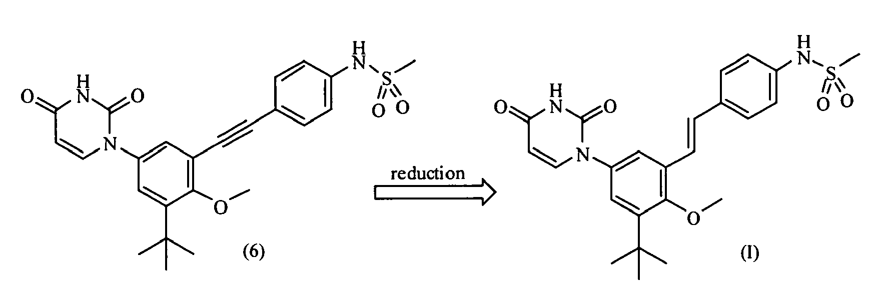

2. Procédé selon la revendication **1,** dans lequel le composé **6** est réduit à l'aide du silane.

3. Procédé selon la revendication **2,** dans lequel le silane est choisi dans le groupe constitué par le triéthylsilane, le phénylsilane, le diphénylsilane, le tripropylsilane, le triphénylsilane, le tribenzylsilane, le 1,1,1,3,5,5,5-heptaméthyltrisiloxane, le tributylsilane, le di-tert-butylsilane, le diéthyloxysilane, le diméthoxysilane, le phényldiethoxyéthylsilane, le diméthylsilane, l'halosilane et le tris(triméthylsilyl)silane.

4. Procédé selon la revendication **1,** dans lequel le composé **6** est réduit à l'aide du disilane.

5. Procédé selon la revendication **4,** dans lequel le disilane est choisi dans le groupe constitué par l'hexaméthyldisilane, l'héxaphényldisilane, le 1,2-diphényltétraméthyldisilane, le 1,2-diméthyl-1,1,2,2,-tétraphéenyldisilane, le 1,1,2,2-tétraméthyldisilane, le 1,2-diéthoxy-1,1,2,2-tétraméthyldisilane, le 1,2-diméthoxy-1,1,2,2-tétraméthyldisilane, et l'hexaméthoxydisilane.

6. Procédé selon l'une quelconque des revendications **1** à **5,** dans lequel le composé **6** est réduit en présence d'un catalyseur.

7. Procédé selon la revendication **6,** dans lequel le catalyseur est un catalyseur au palladium.

8. Procédé selon la revendication **7,** dans lequel le catalyseur est choisi dans le groupe constitué par le tétrakis(triphénylphosphine)palladium (0), le dichlorobis(tri-o-tolylphosphine)palladium (II), l'acétate de palladium (II), le [1,1'-bis (diphénylphosphino)ferrocène]dichloropalladium (II), le tris(dibenzylidèneacétone) dipalladium (0), le dichloro(dibenzylidèneacétone)dipalladium (II), le dichlorotris (cyclohexylphosphine)- palladium (II), le dichlorobis-(triphénylphosphine) palladium (II), et le chloro(η3-allyl)palladium(II) dimère-triphénylphosphine.

9. Procédé selon l'une quelconque des revendications **1** à **8,** dans lequel le composé **6** est réduit en présence d'un solvant.

10. Procédé selon la revendication **9,** dans lequel le solvant est choisi dans le groupe constitué par le térahydrofuran, le diméthylformamide, le diméthylacétamide et le N-méthylpyrrolidone.

11. Procédé selon l'une quelconque des revendications **1** à **10,** dans lequel le composé **6** est préparé par réaction de 1-(3-tert-butyl-5-éthyny1-4-méthoxy-phényl)-pyrimidine-2,4(1H, 3H)-dione (composé **4)** avec le composé **2** choisi dans le groupe constitué de N-(4-iodophényl)méthanesulfonamide (composé **2a**), de N-(4-bromophényl)méthanesulfonamide (composé **2b),** de N-(4-chlorophényl)méthanesulfonamide (composé **2c**), de N-(4-[(arylsulfonyl)oxy]phényl)méthanesulfonamide (composé **2d**), et de N-(4-[(perfluoroalkylsulfonypoxy]phénypméthanesulfonamide (composé **2e**) :

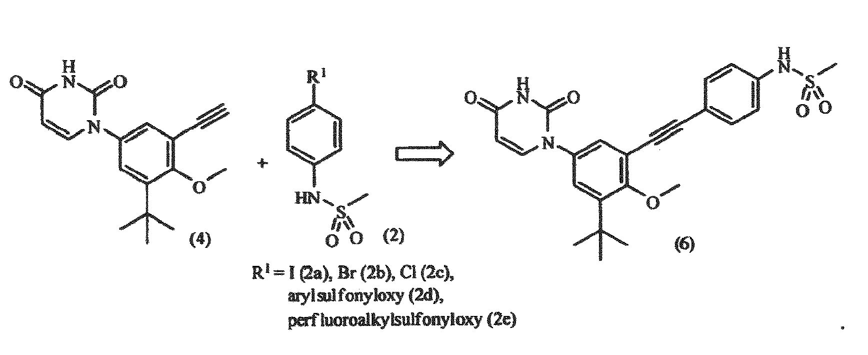

12. Procédé selon la revendication **11,** dans lequel le composé **4** et le composé **2** sont mis en réaction en présence d'une base.

13. Procédé selon l'une quelconque des revendications **11** et **12,** dans lequel le composé **4** et le composé **2** sont mis en réaction en présence d'un solvant.

14. Procédé selon l'une quelconque des revendications **11** à **13,** dans lequel le composé **4** est préparé par :
la réaction d'éthynyltriméthylsilane (≡TMS) (composé **7**) avec le composé 3, choisi dans le groupe constitué de 1-(3-tert-butyl-5-iodo-4-méthoxy-phényl)-pyrimidine-2,4
(1H,3H)-dione (composé **3a**), de 1-(3-bromo-5-tert-butyl-4-méthoxyphényppyrimidine-2,4
(1H,3H)-dione (composé **3b**), et de 1-(3-tert-butyl-5-chloro-4-méthoxy-phényl)-pyrimidine-2,4(1H,3H)-dione (composé 3c) formant ainsi 1-(3-tert-butyl-4-méthoxy-5-((triméthylsilyl)éthynyl)phényl)pyrimidine-2,4(1H,3H)-dione (composé **8**) : 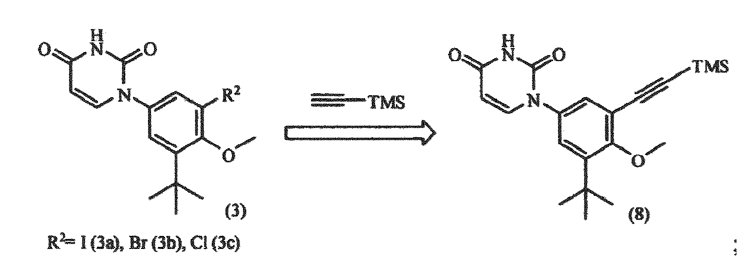 ;et
l'élimination du groupe triméthylsilyle (TMS) formé à partir de 1-(3-tert-butyl-4-méthoxy-5-((triméthylsilypéthynyl)phényl)pyrimidine-2,4(1H,3H)-dione (composé **8**) :

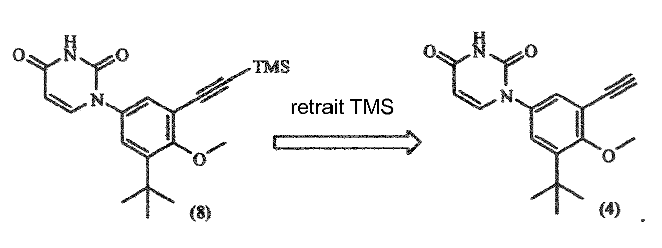

15. Procédé selon l'une quelconque des revendications **1** à **10,** dans lequel le composé **6** est préparé en faisant réagir le N-(4-éthynylphényl)méthanesulfonamide (composé **5**) avec le composé **3** choisi dans le groupe constitué de 1-(3-tert-butyl-5-iodo-4-méthoxy-phényl)-pyrimidine-2,4(1H,3H)-dione (composé **3a**), de 1-(3-bromo-5-tert-butyl-4-méthoxy-phényl)-pyrimidine-2,4(1H,3H)-dione (composé **3b**), et de 1-(3-tert-butyl-5-chloro-4-méthoxyphényppyrimidine-2,4(1H,3H)-dione (composé **3c**) formant ainsi N-(44(3-tert-butyl-5-(2,4-dioxo-3,4-dihydropyrimidine-1(2H)-yl)-2-méthoxyphényl)éthynyl)phényl) méthanesulfonamide (composé **6**) :

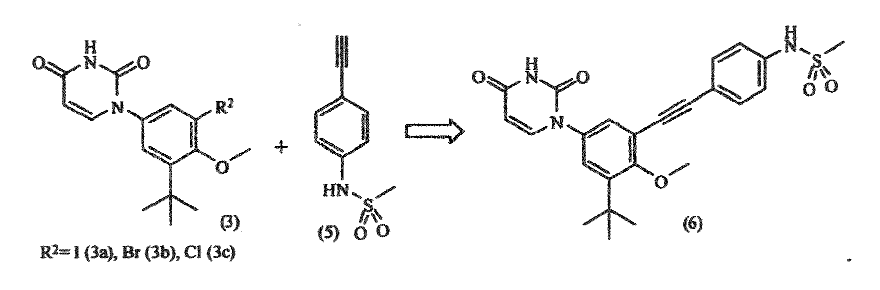

16. Procédé selon la revendication **15,** dans lequel le composé **5** est préparé par :
la réaction d'éthynyltriméthylsilane (≡TMS) (composé **7**) avec le composé **2** choisi dans le groupe constitué de N-(4-iodophényl)méthanesulfonamide (composé **2a**), de N-(4-bromophényl) méthanesulfonamide (composé **2b**), de N-(4 -chlorophényl)méthanesulfonamide (composé **2c**), de N-(4-[(arylsulfonyl)oxy]phényl)méthanesulfonamide (composé **2d**), et de N-(4-[(perfluoroalkylsulfonyl)oxy]phényl)méthanesulfonamide (composé **2e**) formant ainsi N-(4-((triméthylsilyl)éthynyl)phényl) méthanesulfonamide (composé **9**) : 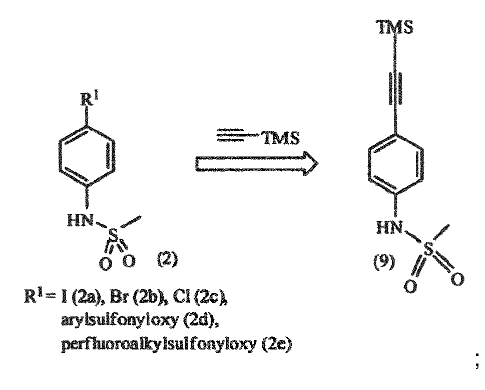 et
l'élimination du groupe triméthylsilyle (TMS) du N-(4-((triméthylsilyl)éthynyl)phényl) méthanesulfonamide formé (composé **9**) :

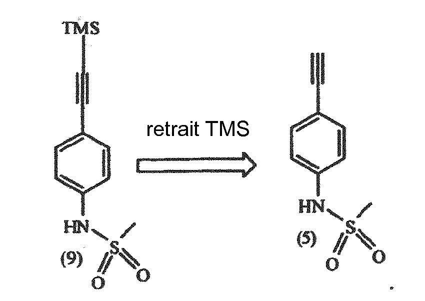

17. Composé ou sel de celui-ci, dans lequel le composé est choisi dans le groupe constitué par les composés répondant à la structure de la formule suivante : 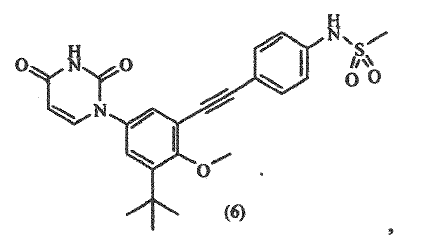 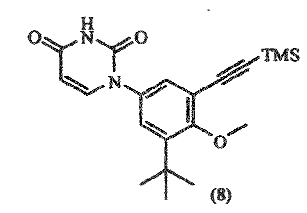 et
